# EUROPÄISCHE PATENTSCHRIFT

(11) **EP 3 909 549 B1**
(45) Veröffentlichungstag und Bekanntmachung des Hinweises auf die Patenterteilung: **26.07.2023**
(21) Anmeldenummer: 21173684.8
(22) Anmeldetag: 12.05.2021
(51) Int. Cl.: A61F 2/28, A61F 2/30, A61F 2/38, A61L 27/06, A61L 27/32, A61L 27/34, A61L 27/56, A61L 27/58

(54) **KNOCHENIMPLANTATSYSTEM**
BONE IMPLANT SYSTEM
SYSTÈME D'IMPLANT OSSEUX

(30) Priorität: 14.05.2020 DE 102020206098
(43) Veröffentlichungstag der Anmeldung: 17.11.2021
(73) Patentinhaber: Aesculap AG, 78532 Tuttlingen (DE)
(72) Erfinder: Stolinski, Elvira, 88605 Meßkirch (DE); Baxmann, Marc, 31177 Harsum (DE); Hoeffgen, Denis, 78324 Engen (DE); Hettich, Georg, 79117 Freiburg (DE); Grupp, Thomas, 78588 Denkingen (DE)
(74) Vertreter: Patentanwälte Ruff, Wilhelm, Beier, Dauster & Partner mbB

(56) Entgegenhaltungen:
- EP-B1- 0 366 945
- WO-A1-2021/155014
- CN-B- 108 158 696
- US-A- 5 211 664
- US-A1- 2007 118 229
- US-A1- 2016 287 391

## Beschreibung

Die Erfindung ist in Anspruch 1 definiert und betrifft ein Knochenimplantatsystem, insbesondere zur Behandlung oder Versorgung von Knochendefekten und/oder zur Verankerung oder Fixierung eines Gelenkimplantats, vorzugsweise Kniegelenkimplantats, sowie ein Knochenimplantat.

Generell stellen komplexe Knochendefekte bei Revisionsoperationen an einem Gelenk eine große Herausforderung dar. Dies gilt insbesondere für die Behandlung oder Versorgung von komplexen tibialen oder femoralen Knochendefekten. Die Behandlung bzw. Versorgung solcher Knochendefekte wird zusätzlich dadurch erschwert, dass die Form und/oder Geometrie von Tibia (Schienbein) und Femur (Oberschenkelknochen) abhängig von der Größe, des Geschlechts sowie der ethnischen Herkunft eines Patienten einer natürlichen Streuung unterliegt.

Bei der Behandlung komplexer Gelenkknochendefekte werden in der Regel zwei vorrangige Ziele verfolgt, nämlich eine stabile Verankerung eines für die Behandlung bzw. Versorgung des Knochendefekts verwendeten Knochenimplantats in einem knöchernen Lager mit hoher Primärstabilität sowie eine Rekonstruktion der ursprünglichen biomechanischen Achsen. Konventionelle Implantate haben in der Regel den Nachteil, dass sie die morphologischen Gegebenheiten, insbesondere die Form und/oder die Geometrie und/oder die Größenverhältnisse, von Knochendefekten, insbesondere tibialen oder femoralen Knochendefekten, nur eingeschränkt abbilden. Eine Folge hiervon ist, dass die Form und/oder die Geometrie und/oder die Größe des Knochendefekts an das jeweilige Implantat angepasst werden muss, um eine optimale kortikale Abstützung zu erzielen. Der hiermit einhergehende (zusätzliche) Knochenverlust erzeugt wiederum ungünstige Ausgangsbedingungen im Hinblick auf etwaige erforderlich werdende Revisionsoperationen.

US 2007/118229 A1 stellt den nächstliegenden Stand der Technik dar und offenbart einen hohlen Grundkörper, an welchen Formkörper variabel angebracht werden können.

Weiterhin offenbaren auch US 5211664 A, CN 108158696 B, US 2016/287391 und WO 2021/155014 A1 Grundkörper mit zugehörigen Formkörpern oder Abstützstrukturen.

### AUFGABE UND LÖSUNG

Aufgabe der Erfindung ist es, ein Knochenimplantatsystem bereitzustellen, welches die eingangs im Zusammenhang von konventionellen Knochenimplantaten beschriebenen Nachteile teilweise oder vollständig vermeidet. Das Knochenimplantatsystem soll insbesondere eine knochenschonende Behandlung oder Versorgung von Knochendefekten ermöglichen und das Risiko von Revisionsoperationen reduzieren.

Diese Aufgabe wird gelöst durch ein Knochenimplantatsystem mit den Merkmalen gemäß unabhängigem Anspruch 1 sowie durch ein Knochenimplantat gemäß Anspruch 14. Bevorzugte Ausgestaltungen der Erfindung sind Gegenstand der abhängigen Ansprüche 2 bis 13 sowie der vorliegenden Beschreibung. Der Wortlaut sämtlicher Ansprüche wird hiermit durch ausdrückliche Bezugnahme zum Inhalt der vorliegenden Beschreibung gemacht.

Die Erfindung betrifft ein Knochenimplantatsystem, vorzugsweise zur Behandlung oder Versorgung von Knochendefekten, insbesondere tibialen und/oder femuralen Knochendefekten, und/oder zur Fixierung oder Verankerung eine Gelenkimplantats, insbesondere Kniegelenkimplantats.

Das Knochenimplantatsystem weist, insbesondere räumlich oder physikalisch voneinander getrennt, Folgendes auf:
- einen Grundkörper, aufweisend ein lasttragendes Material oder bestehend aus einem lasttragenden Material, und
- eine Vielzahl von Formkörpern und/oder eine Abstützstruktur.

Der Grundkörper ist als ein in axialer Richtung beidseitig offener oder geöffneter Hohlkörper gestaltet, d.h. der Grundkörper liegt in Form eines in axialer Richtung beidseitig offenen oder geöffneten Hohlkörpers vor.

Die Formkörper sind derart mit dem Grundkörper, insbesondere stoffschlüssig und/oder formschlüssig und/oder kraftschlüssig, verbindbar, dass die Formkörper, vorzugsweise in radialer Richtung oder außenseitig, von dem Grundkörper abragen oder abstehen.

Die Abstützstruktur ist derart mit dem Grundkörper, insbesondere stoffschlüssig und/oder formschlüssig und/oder kraftschlüssig, verbindbar, dass die Abstützstruktur oder eine Innenfläche davon, insbesondere abschnitts- oder bereichsweise, radial von dem Grundkörper oder einer Außenfläche davon beabstandet ist, vorzugsweise unter Ausbildung eines Hohlraumes oder einer Kavität zwischen dem Grundkörper und der Abstützstruktur.

Die Abstützstruktur ist vorzugsweise dazu ausgebildet, einen kortikalen Knochendefekt, insbesondere des Schienbeins (Tibia), vorzugsweise proximalen Schienbeins (proximale Tibia), oder des Oberschenkelknochens (Femur), vorzugsweise distalen Oberschenkelknochens (distaler Femur), zu umschließen. Alternativ oder in Kombination ist die Abstützstruktur bevorzugt dazu ausgebildet, ein Einbringen eines Knochenersatzmaterials in einen zu behandelnden oder zu versorgenden Knochendefekt zu ermöglichen.Unter dem Ausdruck "Knochenimplantatsystem" soll im Sinne der vorliegenden Erfindung ein Kit oder Set aus einzelnen Komponenten verstanden werden, wobei die Komponenten, insbesondere während eines chirurgischen Eingriffs, zu einem Knochenimplantat, vorzugsweise zur Behandlung oder Versorgung eines tibialen oder femuralen Knochendefektes und/oder zur Fixierung oder Verankerung eines Gelenkimplantats, insbesondere Kniegelenkimplantats, zusammengebaut oder zusammengefügt werden können. Das Set bzw. Kit weist als Komponenten wenigstens einen erfindungsgemäßen Grundkörper sowie eine Vielzahl von erfindungsgemäßen Formkörpern auf. Zusätzlich kann das Kit bzw. Set weitere Komponenten, insbesondere wie nachfolgend näher beschrieben, aufweisen.

Unter dem Ausdruck "Formkörper" sollen im Sinne der vorliegenden Erfindung Körper oder Komponenten des Knochenimplantatsystems verstanden werden, welche dazu ausgebildet sind, eine Fixierung, Verankerung oder Abstützung des Grundkörpers an/in einem Knochendefekt, insbesondere an/in einem tibialen oder femuralen Knochendefekt, zu bewirken. Die Formkörper können im Sinne der vorliegenden Erfindung daher auch als Verankerungskörper, insbesondere zum Verankern des Grundkörpers an/in einem Knochendefekt bezeichnet werden. Die Formkörper des Knochenimplantatsystems können dabei jeweils gleich oder verschieden ausgebildet sein.

Unter dem Ausdruck "Vielzahl von Formkörpern" soll im Sinne der vorliegenden Erfindung wenigstens zwei, insbesondere mehr als zwei, Formkörper, beispielsweise zwei, drei, vier, fünf, sechs, sieben, acht, neun, zehn oder mehr Formkörper, verstanden werden.

Der Ausdruck "Knochendefekt" bedeutet im Sinne der vorliegenden Erfindung einen durch Verlust oder Beschädigung von Knochengewebe, insbesondere Gelenksknochengewebe, vorzugsweise Knie- oder Hüftgelenksknochengewebe, betroffenen Knochenbereich, insbesondere Gelenksknochenbereich, vorzugsweise Knie- oder Hüftgelenksknochenbereich. Der Knochenverlust oder die Knochenbeschädigung kann die Folge einer Knochenfraktur, eines Knochentraumas, einer Nekrose, einer Ausdünnung (beispielsweise infolge von Osteoporose), einer Knochenerkrankung wie Tumorerkrankung, einer fehlenden mechanischen Belastung (sogenanntes Stress Shielding) oder die Folge einer chirurgischen Intervention/Reintervention, insbesondere einer Revision, vorzugsweise nach einem vollständigen Knie- oder Hüftgelenkersatz, sein. Besonders bevorzugt bedeutet der Ausdruck "Knochendefekt" im Sinne der vorliegenden Erfindung einen Schienbein- oder Oberschenkelknochendefekt (tibialer oder femuraler Defekt), insbesondere einen proximalen Schienbeindefekt (proximaler Tibia-Defekt) oder distalen Oberschenkelknochendefekt (distaler Femur-Defekt). Insbesondere kann es sich bei dem Knochendefekt im Sinne der vorliegenden Erfindung um einen sogenannten kavitären Knochendefekt, d.h. um einen Knochendefekt, insbesondere Tibia- oder Femur-Defekt, im Spongiosa-Bereich, oder um einen sogenannten nicht umschlossenen (uncontained) Knochendefekt, insbesondere Tibia- oder Femur-Defekt, handeln, d.h. um einen Knochendefekt, insbesondere Tibia- oder Femur-Defekt, bei dem der kortikale Rand des Knochens, insbesondere der Tibia, bevorzugt proximalen Tibia, oder des Femurs, bevorzugt distalen Femurs, Lücken aufweist.

Der Ausdruck "in vivo abbaubar/in vivo resorbierbar" bedeutet im Sinne der vorliegenden Erfindung in vivo abbaubar oder in vivo resorbierbar.

Der Ausdruck "nicht in vivo abbaubar/nicht in vivo resorbierbar" bedeutet im Sinne der vorliegenden Erfindung nicht in vivo abbaubar oder nicht in vivo resorbierbar.

Unter dem Ausdruck "lasttragendes Material" soll im Sinne der vorliegenden Erfindung ein Material verstanden werden, welches in der Lage ist, in einem Knochen, wie insbesondere Schienbein und/oder Oberschenkelknochen, üblicherweise auftretenden Kräften ohne oder ohne wesentliche Deformation oder Zerstörung zu widerstehen.

Unter dem Ausdruck "in vivo abbaubares Material" soll im Sinne der vorliegenden Erfindung ein Material verstanden werden, welches in einem menschlichen oder tierischen Körper, insbesondere unter der Einwirkung von Enzymen, metabolisiert werden kann. Der Abbau des Materials kann dabei vollständig bis zur Mineralisierung, d.h. Freisetzung von chemischen Elementen und deren Einbau in anorganische Verbindungen, wie beispielsweise Kohlendioxid und/oder Sauerstoff und/oder Ammoniak, verlaufen oder auf der Stufe von abbaustabilen Zwischen- oder Transformationsprodukten stehen bleiben.

Unter dem Ausdruck "in vivo resorbierbares Material" soll im Sinne der vorliegenden Erfindung ein Material verstanden werden, welches in einem menschlichen oder tierischen Körper durch lebende Zellen oder lebendes Gewebe, wie beispielsweise Nieren, aufgenommen werden kann, ohne dass ein Abbau oder ein nennenswerter Abbau des Materials stattfindet.

Unter dem Ausdruck "tierischer Körper" soll im Sinne der vorliegenden Erfindung der Körper eines nicht humanen Säugetiers, wie beispielsweise von Pferd, Kuh, Ziege, Schaf, Schwein oder einem Nagetier, wie beispielsweise Hase, Ratte oder Maus, verstanden werden.

Der Ausdruck "in axialer Richtung" soll im Sinne der vorliegenden Erfindung entlang einer Achse, vorzugsweise entlang einer Längsachse, einer Komponente oder eines Körpers, wie beispielsweise des Grundkörpers und/oder eines optional vorhandenen Trägerkörpers, auf welchen im Folgenden noch näher eingegangen werden wird, des Knochenimplantatsystems bedeuten.

Unter dem Ausdruck "Längsachse" soll im Sinne der vorliegenden Erfindung eine Achse einer Komponente oder eines Körpers, wie beispielsweise des Grundkörpers und/oder eines optional vorhandenen Trägerkörpers, auf welchen im Folgenden noch näher eingegangen werden wird, des Knochenimplantatsystems verstanden werden, welche der Richtung der größten Ausdehnung der Komponente bzw. des Körpers, wie beispielsweise des Grundkörpers und/oder des Trägerkörpers, entspricht.

Der Ausdruck "in radialer Richtung" soll im Sinne der vorliegenden Erfindung von einem Symmetriepunkt, insbesondere Mittelpunkt, und/oder einer Symmetrieachse, insbesondere Mittelachse, einer Komponente oder eines Körpers, wie beispielsweise des Grundkörpers und/oder eines optional vorhandenen Trägerkörpers, auf welchen im Folgenden noch näher eingegangen werden wird, des Knochenimplantatsystems wegzeigend bedeuten.

Der Ausdruck "proximal" soll im Sinne der vorliegenden Erfindung zur Körpermitte zeigend oder näher zur Körpermitte gelegen bedeuten.

Der Ausdruck "distal" soll im Sinne der vorliegenden Erfindung von der Körpermitte weg zeigend oder von der Körpermitte entfernt gelegen bedeuten.

Unter dem Ausdruck "konusförmiger Hohlkörper" soll im Sinne der vorliegenden Erfindung ein Hohlkörper verstanden werden, welcher einen Mantel oder eine Mantelfläche aufweist, wobei der Mantel bzw. die Mantelfläche wenigstens abschnittsweise, insbesondere nur abschnittsweise oder durchgehend, gegenüber einer Längsachse, insbesondere Symmetrieachse, des Hohlkörpers geneigt ist.

Die Erfindung zeichnet sich insbesondere durch nachfolgende Vorteile aus:
- Aufgrund seiner modularen Zusammensetzung (Grundkörper und Vielzahl von Formkörpern) ermöglicht das erfindungsgemäße Knochenimplantatsystem, dass patientenspezifischen Anatomie- und/oder Knochengegebenheiten, insbesondere Knochendefekten, besser Rechnung getragen werden kann. So unterliegen insbesondere die Form und/oder Geometrie von Knochen, wie beispielsweise einer Tibia (Schienbein) und eines Femurs (Oberschenkelknochen), abhängig von Größe, Geschlecht und ethnischer Herkunft der Patienten einer natürlichen Streuung. Durch das erfindungsgemäße Knochenimplantatsystem ist somit im Vergleich zu gattungsgemäßen Implantaten eine patientenindividuellere und mithin optimalere Behandlung und/oder Versorgung von Knochendefekten, insbesondere tibialen und/oder femuralen Knochendefekten, und/oder eine entsprechende Verankerung von Gelenkimplantaten, insbesondere Kniegelenkimplantaten, erzielbar.
- Ferner erlaubt das erfindungsgemäße Knochenimplantatsystem insbesondere aufgrund seiner modularen Zusammensetzung die Behandlung und/oder Versorgung einer Vielzahl unterschiedlicher und insbesondere komplexer Knochendefekte.
- Ferner erlauben die Formkörper mit besonderem Vorteil eine stabile Verankerung des Grundkörpers in einem Knochendefekt und insbesondere eine Überbrückung von etwaigen Fehlstellen zwischen dem Grundkörper und einem Knochendefekt.
- Ferner erlauben die Formkörper vorteilhafterweise die Behandlung oder Versorgung lokaler Knochendefekte, wie beispielsweise einseitiger medialer Knochendefekte.
- Ferner ist von Vorteil, dass das erfindungsgemäße Knochenimplantatsystem eine Anpassung während einer Operation ermöglicht. Dies wiederum ermöglicht eine situative und bedarfsgerechte Behandlung und/oder Versorgung von Knochendefekten und/oder eine entsprechende Verankerung von Gelenkimplantaten.
- Ein weiterer Vorteil besteht darin, dass das erfindungsgemäße Knochenimplantatsystem eine knochenschonende, d.h. knochenverlustreduzierende oder -vermeidende, Behandlung und/oder Versorgung von Knochendefekten und/oder eine entsprechende Verankerung von Gelenkimplantaten erlaubt. Insbesondere kann ein Verlust von natürlichem Knochen durch Abtrag, beispielsweise Bohren und/oder Schleifen, reduziert oder vollständig vermieden werden. Dadurch kann natürliches Knochengewebe in stärkerem Umfang erhalten bleiben.
   Dies stabilisiert zusätzlich eine Fixierung oder Verankerung eines Knochenimplantats, welches durch Zusammenfügen des Grundkörpers sowie der Formkörper erhältlich ist, an/in einem zu behandelnden oder versorgenden Knochendefekt.
- Mithin kann durch das erfindungsgemäße Knochenimplantatsystem in vorteilhafter Weise das Risiko von Revisionsoperationen gesenkt und/oder verbesserte Ausgangsbedingungen für etwaige Revisionsoperationen geschaffen werden.

In Ausgestaltung der Erfindung kann der Grund- oder Hohlkörper einen eckenlosen, insbesondere kreisförmigen, ovalen oder elliptischen, Querschnitt aufweisen.

In weiterer Ausgestaltung der Erfindung kann der Grund- oder Hohlkörper konusförmig gestaltet sein.

Vorzugsweise weist der Grund- oder Hohlkörper in axialer Richtung, insbesondere in Längsrichtung, eine sich verjüngende, insbesondere eine sich kontinuierlich oder diskontinuierlich verjüngende, Querschnittsfläche und/oder einen sich verjüngenden, insbesondere einen sich kontinuierlich oder diskontinuierlich verjüngenden, Innendurchmesser auf.

In weiterer Ausgestaltung der Erfindung kann der Grund- oder Hohlkörper eine poröse, insbesondere offenporige, und/oder mikrostrukturierte Oberfläche aufweisen. Hierdurch kann eine Osteointegration, d.h. ein An- oder Einwachsen von natürlichem Knochengewebe, induziert werden. Dies wiederum begünstigt mit besonderem Vorteil eine stabile Fixierung oder Verankerung des Grundkörpers an/in einem Knochendefekt. Dadurch sind insbesondere verbesserte Sekundärstabilitäten erreichbar. Ferner bewirkt das An- oder Einwachsen von Knochengewebe in vorteilhafter Weise eine Knochendefektverkleinerung.

Bevorzugt weist die poröse, insbesondere offenporige, Oberfläche des Grund- oder Hohlkörpers Poren mit einem Durchmesser, insbesondere mittleren Durchmesser, bestimmt mittels Rasterelektronenmikroskopie und/oder Quecksilber-Porosimetrie, von 10 µm bis 400 µm, insbesondere 50 µm bis 200 µm, bevorzugt 100 µm bis 135 µm, auf. Die in diesem Absatz offenbarten Porendurchmesser sind im Hinblick auf eine Osteointegration des Grund- oder Hohlkörpers besonders vorteilhaft.

Die mikrostrukturierte Oberfläche des Grund- oder Hohlkörpers kann beispielsweise von einer Metallstruktur, insbesondere Metallgitterstruktur, gebildet sein. Vorzugsweise handelt es sich bei der Metallstruktur, insbesondere Metallgitterstruktur, um eine Titanstruktur, insbesondere Titangitterstruktur. Eine entsprechende Titanstruktur ist beispielsweise unter der Bezeichnung Structan^{®} kommerziell erhältlich. Bezüglich weiterer geeigneter Materialien für den Grund- oder Hohlkörper wird auf die noch folgende Beschreibung Bezug genommen.

Unter dem Ausdruck "mikrostrukturierte Oberfläche" soll im Sinne der vorliegenden Erfindung eine Oberfläche mit Strukturelementen, insbesondere in Form von Vertiefungen und/oder Erhebungen, verstanden werden, wobei die Strukturelemente Abmessungen, beispielsweise Tiefen und/oder Höhen, im Mikrometermaßstab besitzen.

Beispielsweise kann die mikrostrukturierte Oberfläche des Grund- oder Hohlkörpers Vertiefungen mit einer Tiefe von 20 µm bis 1000 µm, insbesondere 100 µm bis 600 µm, bevorzugt 300 µm bis 400 µm, und/oder Erhebungen mit einer Höhe von 20 µm bis 1000 µm, insbesondere 100 µm bis 600 µm, bevorzugt 300 µm bis 400 µm, aufweisen.

In weiterer Ausgestaltung der Erfindung kann die Oberfläche des Grund- oder Hohlkörpers von einer Beschichtung des Grund- oder Hohlkörpers gebildet sein. Mit anderen Worten kann es sich bei der Oberfläche des Grund- oder Hohlkörpers in weiterer Ausgestaltung der Erfindung um eine Oberfläche einer Beschichtung handeln, welche den Grund- oder Hohlkörper beschichten oder bedecken kann.

Die Beschichtung kann den Grund- oder Hohlkörper wenigstens teilweise, insbesondere nur teilweise oder vollständig, beschichten oder bedecken. Ferner kann die Beschichtung eine Dicke von 0,01 µm bis 1000 µm, insbesondere 1 µm bis 100 µm, vorzugsweise 10 µm bis 50 µm, aufweisen. Ferner kann die Beschichtung eine Porosität von 20 % bis 90 %, insbesondere 50 % bis 85 %, bevorzugt 60 % bis 80 %, aufweisen. Bevorzugt weist die Beschichtung Titan, d.h. elementares oder metallisches Titan, oder ein Calciumphoshat, vorzugsweise Hydroxylapatit, auf. Beispielsweise kann es sich bei der Beschichtung um eine reine Titanbeschichtung, d.h. um eine aus Titan bestehende Beschichtung, handeln. Eine entsprechende Beschichtung kann beispielsweise mittels eines von der Anmelderin unter der Bezeichnung Plasmapore^{®} durchgeführten Verfahrens hergestellt werden. Hierbei wird reines Titanpulver mittels eines Plasmabeschichtungsprozesses unter Vakuum oder Unterdruck mit einer Dicke von 150 µm und einer Porosität zwischen 35 % und 60 % auf ein zu beschichtendes Substrat aufgebracht. Bezüglich weiterer geeigneter Materialien für die Beschichtung wird auf die im Folgenden noch im Zusammenhang des Grund- oder Hohlkörpers beschriebenen Materialien Bezug genommen.

Ferner kann der Grund- oder Hohlkörper eine Wand- oder Manteldicke, insbesondere eine konstante, d.h. gleichförmige, oder variierende, d.h. ungleichmäßige, Wand- oder Manteldicke, bevorzugt von 1 mm bis 50 mm, insbesondere 5 mm bis 40 mm, vorzugsweise 10 mm bis 20 mm, aufweisen.

Ferner kann der Grund- oder Hohlkörper in axialer Richtung, insbesondere in Längsrichtung, eine Wandung oder einen Mantel mit einer Anzahl von Durchbrüchen oder Aussparungen, d.h. nur einem Durchbruch/eine Aussparung oder einer Vielzahl von Durchbrüchen/Aussparungen, aufweisen. Der Durchbruch/die Aussparung oder die Durchbrüche/Aussparungen kann/können insbesondere u-förmig gestaltet sein. Bevorzugt weist die Wandung oder der Mantel des Grund-oder Hohlkörpers zwei Durchbrüche/Aussparungen, insbesondere zwei gegenüberliegend angeordnete Durchbrüche/Aussparungen, auf. Dadurch kann mit besonderem Vorteil ein Kniegelenksimplantat in das Knochenimplantatsystem positioniert werden. Tibiale Komponenten von Kniegelenksimplantaten besitzen medio-lateral angebrachte "Flügel", um eine stabile Verankerung und eine Rotationsstabilität zu erhalten.

In weiterer Ausgestaltung der Erfindung kann es sich bei dem lasttragenden Material des Grund- oder Hohlkörpers um ein in vivo nicht abbaubares/in vivo nicht resorbierbares Material handeln.

Das in vivo nicht abbaubare/in vivo nicht resorbierbare Material ist vorzugsweise ausgewählt aus der Gruppe bestehend aus Metalle, Legierungen, Keramiken, Kunststoffe und Kombinationen, insbesondere Mischungen, davon.

Das Metall kann insbesondere ausgewählt sein aus der Gruppe bestehend aus Tantal, Titan, Eisen, Zirkonium, Niob, Gold und Platin. Besonders bevorzugt ist Titan.

Die Legierung kann insbesondere ausgewählt sein aus der Gruppe bestehend aus Magnesiumlegierung, Tantallegierung, Titanlegierung, Chrom-Cobalt-Legierung, Chrom-Cobalt-Molybdän-Legierung, Eisenlegierung, Stahl wie Edelstahl, Zirconiumlegierung, Nioblegierung, Goldlegierung, Platinlegierung und Kombinationen, insbesondere Mischungen, davon. Besonders bevorzugt ist eine Titanlegierung. Weiter bevorzugt kann es sich bei der Legierung um die Legierung Ti-6Al-4V, das heißt um eine Titanlegierung, insbesondere hochfeste TitanLegierung, handeln, die aus Titan, 6 Massen-% Aluminium sowie 4 Massen-% Vanadium besteht.

Der Kunststoff kann insbesondere ausgewählt sein aus der Gruppe bestehend aus Polyetherketone, Polyolefine, Polyester, Polyamide, Copolymere und Kombinationen, insbesondere Mischungen (Blends), davon.

Die Polyetherketone können insbesondere ausgewählt sein aus der Gruppe bestehend aus Polyetherketon (PEK), Polyetheretherketon (PEEK), Polyetherketonketon (PEKK), Polyetheretheretherketon (PEEEK), Polyetheretherketonketon (PEEKK), Polyetherketonetherketonketon (PEKEKK), Copolymere davon und Kombinationen, insbesondere Mischungen (Blends), davon. Besonders bevorzugt ist Polyetheretherketon (PEEK).

Die Polyolefine können insbesondere ausgewählt sein aus der Gruppe bestehend aus Polyethylen (PE), Polyethylen niedriger Dichte, Polyethylen hoher Dichte, hochmolekulares Polyethylen (HMWPE), ultrahochmolekulares Polyethylen (UHMWPE), Polypropylen (PP), Polytetrafluorethylen (PTFE), Polyvinylidenfluorid, Polyvinylidenchlorid, Polytetrafluorpropylen, Polyhexafluorpropylen, Copolymere davon und Kombinationen, insbesondere Mischungen (Blends), davon.

Die Polyester können insbesondere ausgewählt sein aus der Gruppe bestehend aus Polyethylenterephthalat, Polypropylenterephthalat, Polybutylenterephthalat, Copolymere und Kombinationen, insbesondere Mischungen (Blends), davon.

In weiterer Ausgestaltung der Erfindung können die Formkörper oder nur ein Teil der Formkörper eine poröse, insbesondere offenporige, und/oder mikrostrukturierte Oberfläche aufweisen.

Insbesondere kann die poröse, insbesondere offenporige, Oberfläche der Formkörper Poren mit einem Durchmesser, insbesondere mittleren Durchmesser, bestimmt mittels Rasterelektronenmikroskopie und/oder Quecksilber-Porosimetrie, von 50 µm bis 900 µm, insbesondere 150 µm bis 700 µm, besonders bevorzugt 300 µm bis 400 µm, aufweisen. Die mikrostrukturierte Oberfläche der Formkörper kann Vertiefungen mit einer Tiefe von 50 µm bis 900 µm, insbesondere 150 µm bis 700 µm, bevorzugt 300 µm bis 400 µm, und/oder Erhebungen mit einer Höhe von 50 µm bis 900 µm, insbesondere 150 µm bis 700 µm, bevorzugt 300 µm bis 400 µm, aufweisen. Dadurch ist mit besonderem Vorteil eine Osteointegration und abhängig vom Material der Formkörper auch eine biologische Rekonstruktion eines zu behandelnden oder versorgenden Knochendefekts erzielbar. Ferner ist hierdurch mit besonderem Vorteil eine Verkleinerung eines Knochendefekts erreichbar.

Ferner kann die poröse, insbesondere offenporige, Oberfläche der Formkörper oder eines Teils der Formkörper von einer Beschichtung der Formkörper gebildet sein. Die Beschichtung kann die Formkörper wenigstens teilweise, insbesondere nur teilweise oder vollständig, beschichten oder bedecken. Ferner kann die Beschichtung eine Dicke von 0,01 µm bis 1000 µm, insbesondere 1 µm bis 100 µm, vorzugsweise 10 µm bis 50 µm, aufweisen. Ferner kann die Beschichtung eine Porosität von 20 % bis 90 %, insbesondere 50 % bis 85 %, bevorzugt 50 % bis 70 %, aufweisen. Bezüglich weiterer geeigneter Materialien für die Beschichtung wird auf die im Folgenden noch im Zusammenhang der Formkörper beschriebenen Materialien Bezug genommen.

Ferner können die Formkörper eine Dicke, d.h. Wanddicke oder Höhe, insbesondere eine konstante, d.h. gleichförmige, oder variierende, d.h. ungleichmäßige, Dicke, d.h. Wanddicke oder Höhe, von 1 mm bis 30 mm, insbesondere 5 mm bis 20 mm, vorzugsweise 8 mm bis 15 mm, aufweisen.

Weiter sind die Formkörper vorzugsweise als Flächengebilde gestaltet, d.h. die Formkörper weisen vorzugsweise eine Länge und eine Breite auf, welche größer sind als eine Dicke (Höhe) der Formkörper. Bevorzugt sind die Formkörper platten- und/oder scheibenförmig, insbesondere quaderförmig, gestaltet. Insbesondere können die Formkörper jeweils zwei gegenüberliegend angeordnete Hauptflächen aufweisen, welche jeweils von zwei gegenüberliegend angeordneten Querseitenflächen sowie von zwei gegenüberliegend angeordneten Längsseitenflächen begrenzt sind. Grundsätzlich können die Querseitenflächen plan, d.h. eben oder nicht gewölbt, und/oder gewölbt, insbesondere nach außen gewölbt, sein. Weiter können die Längsseitenflächen grundsätzlich plan, d.h. eben oder nicht gewölbt, und/oder gewölbt, insbesondere nach außen gewölbt, sein. Zum Beispiel können die Formkörper jeweils eine gewölbte, insbesondere nach außen gewölbte, Längsseitenfläche und im Übrigen plane Seitenflächen, d.h. eine plane Längsseitenfläche und zwei plane Querseitenflächen, aufweisen.

Abhängig von dem zu behandelnden oder versorgenden Knochendefekt können die Formkörper auch andere Designs oder Formen aufweisen. Beispielsweise können die Formkörper eine spitz zulaufende Form oder eine Flügelform, insbesondere ähnlich dem Vega Tibia Plateau, besitzen. Alternativ oder in Kombination können die Formkörper einen eckenlosen, insbesondere runden, ovalen oder elliptischen, oder polygonen, insbesondere dreieckigen, viereckigen (zum Beispiel rechteckigen), T-förmigen oder V-förmigen, Querschnitt aufweisen.

Ferner können die Formkörper oder ein Teil der Formkörper eine glatte Oberfläche, d.h. eine Oberfläche ohne Vertiefungen und/oder Erhebungen, aufweisen.

In weiterer Ausgestaltung der Erfindung können die Formkörper oder ein Teil der Formkörper und/oder die Abstützstruktur ein in vivo abbaubares/in vivo resorbierbares Material aufweisen oder die Formkörper und/oder die Abstützstruktur können aus einem in vivo abbaubaren/in vivo resorbierbaren Material bestehen. Dadurch ist mit besonderem Vorteil eine biologische Rekonstruktion und insbesondere sukzessive Verkleinerung eines Knochendefekts realisierbar. Das in vivo abbaubare/in vivo resorbierbare Material ist vorzugsweise ausgewählt aus der Gruppe bestehend aus Metalle, Metalllegierungen, Polyhydroxyalkanoate, Polycaprolacton, Poly-p-dioxanon (Poly-1,4-dioxan-2-on), Polymethylenterephthalat, Calciumphosphate, Calciumsulfate, Calciumoxid (CaO), Calciumhydroxid (Ca(OH)₂), Calciumcarbonat (CaCO₃), Calciumcitrat, Calciumlactat, Calciumacetat, Calciumtartrat, Calciumchlorid (CaCl₂), Magnesiumphosphate, Magnesiumsulfate, Magnesiumhydroxid (Mg(OH)₂), Magnesiumhydroxidcarbonat (beispielsweise als 4 MgCO₃ x Mg(OH)₂ x 5 H₂O), Magnesiumoxid (MgO), Magnesiumcitrate (Mg₃(C₆H₅O₇)₂) oder Mg(C₆H₆O₇)), Calcium-Magnesiumcarbonat (CaMg(CO₃)₂) und Kombinationen, insbesondere Mischungen, davon.

Bei den Metallen handelt es sich vorzugsweise um Magnesium.

Bei den Metalllegierungen kann es sich insbesondere um Magnesiumlegierungen, beispielsweise um eine unter der Bezeichnung "Magnezix^{®}" oder "WE 43" kommerziell erhältliche Magnesiumlegierung, handeln. Die unter der Bezeichnung "Magnezix^{®}" kommerziell erhältliche Magnesiumlegierung besteht aus Magnesium, Zirconium und Metallen der seltenen Erden, wobei die Magnesiumlegierung einen Magnesiumanteil von 90 % oder mehr besitzen kann.

Die Polyhydroxyalkanoate können insbesondere ausgewählt sein aus der Gruppe bestehend aus Polylactid, Polyglycolid, Poly-3-hydroxybutyrat, Poly-4-hydroxybutyrat, Copolymere davon und Kombinationen, insbesondere Mischungen (Blends), davon.

Die Calciumphosphate können insbesondere ausgewählt sein aus der Gruppe bestehend aus Tricalciumphosphat (TCP) wie α-Tricalciumphosphat (α-TCP) und/oder β-Tricalciumphosphat (β-TCP), Apatite wie Hydroxylapatit (HA), Calcium-defizientes Hydroxylapatit (CdHA), substituiertes Hydroxylapatit, nicht stöchiometrisches Hydroxylapatit, nanoskaliges Hydroxylapatit, Tetracalciumphosphat (TTCP), Monocalciumphosphat-Monohydrat (MCPM), Monocalciumphosphat-Anhydrid (MCPA), Dicalciumphosphat-Anhydrid (DCPA), Dicalciumphosphat-Dihydrat (DCPD), Octacalciumphosphat (OCP), amorphes Calciumphosphat (ACP), Calciumglycerophosphat und Kombinationen, insbesondere Mischungen, davon.

Die Calciumsulfate können insbesondere ausgewählt sein aus der Gruppe bestehend aus Calciumsulfat (CaSO₄), Calciumsulfat-Hemihydrat (CaSO₄ x 0.5 H₂O), Calciumsulfat-Dihydrat (CaSO₄ x 2 H₂O) und Kombinationen, insbesondere Mischungen, davon.

Die Magnesiumphosphate können insbesondere ausgewählt sein aus der Gruppe bestehend aus Magnesiumhydrogenphosphat (MgHPO₄) in Form der Hydrate oder als wasserfreie Substanz, Trimagnesiumphosphat (Mg₃(PO₄)₂), Magnesiumdihydrogenphosphat (Mg(H₂PO₄)₂) in Form der Hydrate oder als wasserfreie Substanz, Magnesiumglycerophosphat und Kombinationen, insbesondere Mischungen, davon.

Besonders bevorzugt handelt es sich bei dem in vivo abbaubaren/in vivo resorbierbaren Material um Tricalciumphosphat. Das Tricalciumphosphat kann in kristalliner Form vorliegen. Insbesondere kann das Tricalciumphosphat eine Kristallinität von 50 % bis 99 %, insbesondere 75 % bis 95 %, aufweisen. Ferner kann es sich bei dem Tricalciumphosphat um ein mikrokristallines Tricalciumphosphat, d.h. um Tricalciumphosphat mit Kristalliten, welche wenigstens eine Abmessung oder Dimension im Mikrometerbereich, insbesondere in einem Bereich von > 0,5 µm, insbesondere 0,6 µm bis 500 µm, bevorzugt 0,6 µm bis 100 µm, aufweisen, handeln. Bei der wenigstens einen Abmessung bzw. Dimension kann es sich um die Länge und/oder die Breite und/oder die Dicke bzw. Höhe und/oder den Durchmesser, insbesondere mittleren Durchmesser, der Kristallite handeln. Alternativ kann es sich bei dem Tricalciumphosphat um ein nanokristallines Tricalciumphosphat, d.h. um Tricalciumphosphat mit Kristalliten, welche wenigstens eine Abmessung oder Dimension im Nanometerbereich, insbesondere in einem Bereich von 0,1 nm bis 500 nm, bevorzugt 0,1 nm bis 100 nm, aufweisen, handeln. Bei der wenigstens einen Abmessung bzw. Dimension kann es sich um die Länge und/oder die Breite und/oder die Dicke bzw. Höhe und/oder den Durchmesser, insbesondere mittleren Durchmesser, der Kristallite handeln. Alternativ kann es sich bei dem Tricalciumphosphat um ein makrokristallines Tricalciumphosphat handeln. Alternativ kann das Tricalciumphosphat in amorpher Form vorliegen. Weiter kann es sich bei dem Tricalciumphosphat um ein phasenreines Tricalciumphosphat handeln. Unter dem Ausdruck "phasenrein" soll an dieser Stelle insbesondere phasenrein im Sinne einer einschlägigen Vorschrift, vorzugsweise nach ASTM F1088, verstanden werden. Ferner kann das Tricalciumphosphat eine Porosität < 50 %, insbesondere < 20 %, vorzugsweise < 15 %, aufweisen. Alternativ kann das Tricalciumphosphat nicht porös ausgebildet sein. Vorzugsweise ist das Tricalciumphosphat ausgewählt aus der Gruppe bestehend aus α-Tricalciumphosphat (α-TCP), β-Tricalciumphosphat (β-TCP) und eine Mischung davon. Insbesondere bevorzugt handelt es sich bei dem Tricalciumphosphat um ein gesintertes Tricalciumphosphat, vorzugsweise ausgewählt aus der Gruppe bestehend aus gesintertes α-Tricalciumphosphat (gesintertes α-TCP), gesintertes β-Tricalciumphosphat (gesintertes β-TCP) und eine Mischung davon.

Alternativ kann es sich bei dem in vivo abbaubaren/in vivo resorbierbaren Material insbesondere um Hydroxylapatit handeln. Der Hydroxylapatit kann in kristalliner Form vorliegen. Insbesondere kann der Hydroxylapatit eine Kristallinität von 50 % bis 99 %, insbesondere 75 % bis 95 %, aufweisen. Ferner kann es sich bei dem Hydroxylapatit um einen mikrokristallinen Hydroxylapatit, d.h. um einen Hydroxylapatit mit Kristalliten, welche wenigstens eine Abmessung oder Dimension im Mikrometerbereich, insbesondere in einem Bereich von > 0,5 µm, insbesondere 0,6 µm bis 500 µm, bevorzugt 0,6 µm bis 100 µm, aufweisen, handeln. Bei der wenigstens einen Abmessung bzw. Dimension kann es sich um die Länge und/oder die Breite und/oder die Dicke bzw. Höhe und/oder den Durchmesser, insbesondere mittleren Durchmesser, der Kristallite handeln. Alternativ kann es sich bei dem Hydroxylapatit um einen nanokristallinen Hydroxylapatit, d.h. um einen Hydroxylapatit mit Kristalliten, welche wenigstens eine Abmessung oder Dimension im Nanometerbereich, insbesondere in einem Bereich von 0,1 nm bis 500 nm, bevorzugt 0,1 nm bis 100 nm, aufweisen, handeln. Bei der wenigstens einen Abmessung bzw. Dimension kann es sich um die Länge und/oder die Breite und/oder die Dicke bzw. Höhe und/oder den Durchmesser, insbesondere mittleren Durchmesser, der Kristallite handeln. Alternativ kann es sich bei dem Hydroxylapatit um ein makrokristallines Hydroxylapatit handeln. Weiter kann es sich bei dem Hydroxylapatit um einen phasenreinen Hydroxylapatit handeln. Unter dem Ausdruck "phasenrein" soll in diesem Zusammenhang insbesondere phasenrein im Sinne einer einschlägigen Vorschrift, vorzugsweise nach ASTM F1185, verstanden werden. Ferner kann der Hydroxylapatit eine Porosität < 50 %, insbesondere < 20 %, vorzugsweise < 15 %, aufweisen. Durch eine geringe Porosität kann eine hohe mechanische Stabilität erreicht werden. Alternativ kann der Hydroxylapatit nicht porös ausgebildet sein. Bei dem Hydroxylapatit kann es sich ferner insbesondere um einen gesinterten Hydroxylapatit handeln.

Alternativ können die Formkörper oder ein Teil der Formkörper und/oder die Abstützstruktur ein in vivo nicht abbaubares/in vivo nicht resorbierbares Material aufweisen oder aus einem solchen Material bestehen. Bezüglich geeigneter in vivo nicht abbaubarer/in vivo nicht resorbierbarer Materialien wird auf die vorangegangenen im Zusammenhang des Grund- oder Hohlkörpers erwähnten Materialien Bezug genommen.

Ferner können die Formkörper oder ein Teil der Formkörper und/oder die Abstützstruktur sowohl ein in vivo abbaubares/in vivo resorbierbares Material als auch ein in vivo nicht abbaubares/in vivo nicht resorbierbares Material aufweisen oder sowohl aus einem in vivo abbaubaren/in vivo resorbierbaren Material als auch aus einem in vivo nicht abbaubaren/in vivo nicht resorbierbaren Material bestehen. Bezüglich geeigneter in vivo abbaubarer/in vivo resorbierbarer Materialien sowie vivo nicht abbaubarer/in vivo nicht resorbierbarer Materialien wird vollständig auf die in der bisherigen Beschreibung offenbarten Materialien Bezug genommen.

In weiterer Ausgestaltung der Erfindung kann der Grund- oder Hohlkörper Nuten aufweisen, d.h. längliche Vertiefungen, und/oder Schlitze, d.h. Spalten oder längliche, d.h. längserstreckte, Öffnungen oder Langlöcher, zum formschlüssigen Einsetzen der Formkörper.

Vorzugsweise erstrecken sich die Nuten und/oder Schlitze entlang der Längsachse des Grund- oder Hohlkörpers.

Die Nuten können eine Länge von 10 mm bis 50 mm, insbesondere 15 mm bis 40 mm, bevorzugt 20 mm bis 30 mm, aufweisen. Ferner können die Nuten einen Innendurchmesser von von 0,5 mm bis 20 mm, insbesondere 1 mm bis 10 mm, bevorzugt 2 mm bis 5 mm, aufweisen. Ferner können die Nuten einen planen, d.h. ebenen oder nicht gewölbten, oder gewölbten, insbesondere konvexen oder konkaven, Boden aufweisen.

Ferner können die Nuten beispielsweise als Schwalbenschwanznuten und/oder T-Nuten ausgebildet sein.

Ferner können die Schlitze eine Länge von 10 mm bis 50 mm, insbesondere 15 mm bis 40 mm, bevorzugt 20 mm bis 30 mm, aufweisen. Ferner können die Schlitze einen Innendurchmesser von 0,5 mm bis 20 mm, insbesondere 1 mm bis 10 mm, bevorzugt 2 mm bis 5 mm, aufweisen. Bevorzugt sind die Nuten und/oder Schlitze in axialer Richtung, insbesondere in Längsrichtung, des Grund-oder Hohlkörpers offen oder geöffnet, insbesondere einseitig offen oder geöffnet. Dadurch kann mit besonderem Vorteil das formschlüssige Einsetzen der Formkörper erleichtert werden.

Ferner können die Nuten und/oder Schlitze in axialer Richtung, insbesondere in Längsrichtung, des Grund- oder Hohlkörpers eine sich verjüngende, insbesondere eine sich kontinuierlich oder diskontinuierlich verjüngende, Querschnittsfläche und/oder einen sich in axialer Richtung, insbesondere in Längsrichtung, des Grund- oder Hohlkörpers verjüngenden, insbesondere kontinuierlich oder diskontinuierlich verjüngenden, Innendurchmesser aufweisen. Dadurch kann das formschlüssige Einsetzen der Formkörper (ebenfalls) optimiert werden.

Alternativ oder in Kombination können die Nuten und/oder Schlitze in Querrichtung des Grund-oder Hohlkörpers, d.h. in Tiefenrichtung der Nute und/oder Schlitze, einen sich verjüngenden, insbesondere kontinuierlich oder diskontinuierlich verjüngenden, Innendurchmesser aufweisen. Ferner können die Nuten und/oder Schlitze eine Länge aufweisen, welcher kleiner ist als eine Länge des Grund- oder Hohlkörpers.

Der Grund- oder Hohlkörper gemäß der vorliegenden Erfindung weist sich entlang der Längsachse des Grund- oder Hohlkörpers erstreckende Sollbruchlinien auf, welche nach einem Brechen sich entlang der Längsachse des Grund- oder Hohlkörpers erstreckende Schlitze d.h. Spalten oder längliche Öffnungen oder Langlöcher, freigeben. Hierdurch kann mit besonderem Vorteil je nach Bedarf eine unterschiedliche Anzahl von sich entlang der Längsachse des Grund- oder Hohlkörpers erstreckenden Schlitzen zum formschlüssigen Einsetzen der Formkörper freigeben werden. Dadurch wird mit besonderem Vorteil eine weitere einfache und mithin komplikationslose Möglichkeit für eine patientenindividuelle Behandlung oder Versorgung von Knochendefekten geschaffen. Die Sollbruchlinien können beispielsweise als Perforationen, Kerben oder Ritzspuren gestaltet sein.

In weiterer Ausgestaltung der Erfindung können die Formkörper oder ein Teil der Formkörper jeweils wenigstens eine Sollbruchlinie aufweisen, insbesondere nur eine Sollbruchlinie oder eine Vielzahl von Sollbruchlinien.

Die Sollbruchlinie/Sollbruchlinien kann/können sich insbesondere in Längs- und/oder Querrichtung der Formkörper erstrecken. Weiter können die Sollbruchlinien beispielsweise als Perforationen, Kerben oder Ritzspuren gestaltet sein. Dadurch kann der modulare Charakter und mithin die bereits an sich vielseitige Verwendungsmöglichkeit des erfindungsgemäßen Knochenimplantatsystems zusätzlich erhöht werden. Insbesondere können die Formkörper bei dieser Ausgestaltung der Erfindung variabel gekürzt werden. Dadurch können kavitäre Knochendefekte sowie nicht umschlossene Knochendefekte versorgt oder behandelt werden.

Die Abstützstruktur kann vorteilhafterweise an die patientenspezifische Knochenmorphologie angepasst und insbesondere fixiert werden.

Die Abstützstruktur kann beispielsweise als Vollmaterial oder als ununterbrochenes Flächengebilde, d.h. als durchgehendes beziehungsweise keine Unterbrechungen, wie beispielsweise Löcher, Poren, Perforationen oder dergleichen, aufweisendes Material oder Flächengebilde, gestaltet sein. Alternativ kann die Abstützstruktur als Gitter, insbesondere Lochgitter, vorzugsweise metallisches Lochgitter, gestaltet sein. Alternativ kann die Abstützstruktur in Form eines textilen Flächengebildes, insbesondere in Form eines Gewebes, vorzugsweise flexiblen Gewebes, oder Gewirkes oder Vlieses gestaltet sein.

Ferner kann die Abstützstruktur eine Wanddicke, insbesondere eine konstante, d.h. gleichförmige, oder variierende, d.h. ungleichmäßige, Wanddicke, bevorzugt von 0,2 mm bis 10 mm, insbesondere 1 mm bis 8 mm, vorzugsweise 2 mm bis 4 mm, aufweisen.

In weiterer Ausgestaltung der Erfindung kann das Knochenimplantatsystem ferner einen Trägerkörper aufweisen.

Unter dem Ausdruck "Trägerkörper" soll im Sinne der vorliegenden Erfindung ein Körper oder eine Komponente verstanden werden, welche dazu ausgebildet ist, eine Trägerung der Formkörper zu bewirken, d.h. als Träger für die Formkörper zu dienen oder zu wirken. Bevorzugt sind die Formkörper und der Trägerkörper, vorzugsweise stoffschlüssig, miteinander unter Ausbildung eines Klammerkörpers verbindbar oder verbunden. Beispielsweise können der Trägerkörper und die Formkörper miteinander verklebt oder verschweißt sein oder mittels eines generativen Fertigungsverfahrens miteinander verbunden sein. Bevorzugt ragen die Formkörper, vorzugsweise in radialer Richtung oder außenseitig, vom Trägerkörper ab. Besonders bevorzugt ist der Klammerkörper in axialer Richtung, insbesondere in Längsrichtung, des Grund- oder Hohlkörpers auf eine Wandung oder einen Mantel des Grund-oder Hohlkörpers aufsteckbar oder aufsetzbar, insbesondere wobei die Wandung oder der Mantel des Grund- oder Hohlkörpers beidseitig wenigstens abschnittsweise, insbesondere nur abschnittsweise oder vollständig, von dem Klammerkörper eingefasst oder eingeklemmt wird. Besonders bevorzugt wird die Wandung oder der Mantel des Grund- oder Hohlkörpers auf einer Seite von einer Außenseite oder -fläche des Trägerkörpers und auf einer gegenüberliegenden Seite von der Außenseite oder -fläche des Trägerkörpers zugewandten Längsseitenflächen der Formkörper eingefasst oder eingeklemmt.

In weiterer Ausgestaltung der Erfindung kann der Trägerkörper formkomplementär zu dem Grund-oder Hohlkörper oder einem Teil davon, insbesondere zu einer Innenkontur oder einem Innenkonturabschnitt des Grund- oder Hohlkörpers, gestaltet sein.

Mit anderen Worten kann der Trägerkörper in weiterer Ausgestaltung der Erfindung eine Form aufweisen, welche einer Form des Grund- oder Hohlkörpers oder einem Teil davon entspricht. Insbesondere kann der Trägerkörper (ebenfalls) als ein in axialer Richtung, insbesondere in Längsrichtung, beidseitig offener oder geöffneter Hohlkörper, vorzugsweise konusförmiger Hohlkörper, gestaltet sein. Ferner kann der Trägerkörper einen eckenlosen, insbesondere kreisförmigen, ovalen oder elliptischen, Querschnitt aufweisen. Alternativ oder in Kombination kann der Trägerkörper in axialer Richtung, insbesondere in Längsrichtung, eine sich verjüngende, insbesondere eine sich kontinuierlich oder diskontinuierlich verjüngende, Querschnittsfläche und/oder einen sich verjüngenden, insbesondere einen sich kontinuierlich oder diskontinuierlich verjüngenden, Innendurchmesser aufweisen. Ferner kann der Trägerkörper in axialer Richtung, insbesondere in Längsrichtung, eine Wandung oder einen Mantel mit einer Anzahl von Durchbrüchen oder Aussparungen, d.h. nur einen Durchbruch/eine Aussparung oder eine Vielzahl von Durchbrüchen/Aussparungen, aufweisen. Der Durchbruch/die Aussparung bzw. die Durchbrüche/Aussparungen kann/können insbesondere u-förmig gestaltet sein. Bevorzugt weist die Wandung oder der Mantel des Trägerkörpers zwei Durchbrüche/Aussparungen, insbesondere zwei gegenüberliegend angeordnete Durchbrüche/Aussparungen, auf.

Ferner kann der Trägerkörper ein in vivo nicht abbaubares/in vivo nicht resorbierbares Material und/oder ein in vivo abbaubares/in vivo resorbierbares Material aufweisen oder aus einem in vivo nicht abbaubaren/in vivo nicht resorbierbaren Material und/oder in vivo abbaubaren/in vivo resorbierbaren Material bestehen. Bezüglich geeigneter in vivo nicht abbaubarer/in vivo nicht resorbierbarer Materialien und/oder in vivo abbaubarer/in vivo resorbierbarer Materialien wird vollständig auf die entsprechenden in der bisherigen Beschreibung offenbarten Materialien Bezug genommen.

In weiterer Ausgestaltung der Erfindung kann der Träger- oder Klammerkörper einen Kragenabschnitt aufweisen, an welchem die Formkörper angefügt oder fixiert, vorzugsweise stoffschlüssig angefügt oder fixiert, beispielsweise angeklebt oder angeschweißt, sind. Der Kragenabschnitt erstreckt sich vorzugsweise in Umfangsrichtung des Träger- oder Klammerkörpers und/oder kragt vorzugsweise in radialer Richtung des Träger- oder Klammerkörpers aus. Dadurch kann mit besonderem Vorteil eine Beabstandung der Formkörper zu dem Trägerkörper geschaffen werden, wodurch ein besonders einfaches und komplikationsloses formschlüssiges Zusammenfügen von Grund- und Klammerkörper durch Aufstecken des Klammerkörpers in axialer Richtung auf die Wandung des Grund- oder Hohlkörpers erzielbar ist.

In weiterer Ausgestaltung der Erfindung kann der Klammerkörper im Längsschnitt u-förmig gestaltet sein.

Ferner kann das Knochenimplantatsystem ein Knochenersatzmaterial, vorzugsweise ein partikuläres, insbesondere pulver- oder granulatförmiges, Knochenersatzmaterial aufweisen. Das Knochenersatzmaterial weist vorzugsweise ein in vivo abbaubares/in vivo resorbierbares Material, insbesondere ausgewählt aus der Gruppe bestehend aus Metalle wie Magnesium, Polymere wie Polycaprolacton, Calciumphosphate wie β-Tricalciumphosphat und/oder Hyaluronsäure und Mischungen von wenigstens zwei der vorgenannten in vivo abbaubaren/in vivo resorbierbaren Materialien, und/oder eine Trägermatrix auf. Die Trägermatrix kann ein wasserlösliches Polymer, wie beispielsweise Polyethylenglykol, und/oder ein Protein, vorzugsweise Strukturprotein, insbesondere ausgewählt aus der Gruppe bestehend aus Collagen, Elastin, Retikulin, Fibronektin, Gelatine und Mischungen von wenigstens zwei der vorgenannten Proteine, insbesondere Strukturproteine, sein.

Alternativ oder in Kombination kann das Knochenimplantatsystem ferner ein Bindemittel aufweisen.

Die Erfindung betrifft weiterhin ein Knochenimplantat, vorzugsweise zur Behandlung oder Versorgung von Knochendefekten, insbesondere tibialen und/oder femuralen Knochendefekten, und/oder zur Fixierung oder Verankerung eine Gelenkimplantats, insbesondere Kniegelenkimplantats.

Das Knochenimplantat ist hergestellt oder herstellbar aus einem erfindungsgemäßen Knochenimplantatsystem oder durch Verbinden, insbesondere formschlüssiges Verbinden und/oder kraftschlüssiges Verbinden und/oder stoffschlüssiges Verbinden, eines Grundkörpers, welcher ein lasttragendes Material aufweist oder aus einem lasttragenden Material besteht, und einer Vielzahl von Formkörpern und/oder einer Abstützstruktur. Der Grundkörper ist als ein in axialer Richtung beidseitig offener Hohlkörper gestaltet. Die Formkörper sind derart mit dem Grundkörper, insbesondere stoffschlüssig und/oder formschlüssig und/oder kraftschlüssig, verbunden, dass die Formkörper in radialer Richtung von dem Grundkörper abstehen. Die Abstützstruktur ist derart mit dem Grundkörper, insbesondere stoffschlüssig und/oder formschlüssig und/oder kraftschlüssig, verbunden, dass die Abstützstruktur oder eine Innenfläche davon, insbesondere abschnitts- oder bereichsweise, radial von dem Grundkörper oder einer Außenfläche davon beabstandet ist, vorzugsweise unter Ausbildung eines Hohlraumes oder einer Kavität zwischen dem Grundkörper und der Abstützstruktur.

Bezüglich weiterer Merkmale und Vorteile des Knochenimplantats, insbesondere des Grundkörpers sowie der Vielzahl von Formkörpern und/oder der Abstützstruktur, wird vollständig auf die bisherige Beschreibung Bezug genommen. Die dort insbesondere in Bezug auf den Grundkörper, die Vielzahl von Formkörpern und/oder die Abstützstruktur sowie die weiteren optionalen Komponenten/Körper (wie Trägerkörper und Klammerkörper) beschriebenen Merkmale und Vorteile gelten sinngemäß auch für das Knochenimplantat.

Die Erfindung betrifft ferner ein weiteres Knochenimplantat, vorzugsweise zur Behandlung oder Versorgung von Knochendefekten, insbesondere tibialen und/oder femuralen Knochendefekten, und/oder zur Fixierung oder Verankerung eine Gelenkimplantats, insbesondere Kniegelenkimplantats.

Das Knochenimplantat weist Folgendes auf:
- einen Grundkörper, aufweisend ein lasttragendes Material oder bestehend aus einem lasttragenden Material, und
- eine Vielzahl von Formkörpern und/oder eine Abstützstruktur.

Der Grundkörper ist als ein in axialer Richtung beidseitig offener oder geöffneter Hohlkörper gestaltet, d.h. der Grundkörper liegt in Form eines in axialer Richtung beidseitig offenen oder geöffneten Hohlkörpers vor.

Die Formkörper sind derart mit dem Grundkörper, insbesondere stoffschlüssig und/oder formschlüssig und/oder kraftschlüssig, verbunden, dass die Formkörper, vorzugsweise in radialer Richtung oder außenseitig, von dem Grundkörper abragen oder abstehen.

Die Abstützstruktur ist derart mit dem Grundkörper, insbesondere stoffschlüssig und/oder formschlüssig und/oder kraftschlüssig, verbunden, dass die Abstützstruktur oder eine Innenfläche davon, insbesondere abschnitts- oder bereichsweise, radial von dem Grundkörper oder einer Außenfläche davon beabstandet ist, vorzugsweise unter Ausbildung eines Hohlraumes oder einer Kavität zwischen dem Grundkörper und der Abstützstruktur.

Bezüglich weiterer Merkmale und Vorteile des Knochenimplantats, insbesondere des Grundkörpers sowie der Vielzahl von Formkörpern und/oder der Abstützstruktur, wird vollständig auf die bisherige Beschreibung Bezug genommen. Die dort insbesondere in Bezug auf den Grundkörper, die Vielzahl von Formkörpern und/oder die Abstützstruktur sowie die weiteren optionalen Komponenten/Körper (wie Trägerkörper und Klammerkörper) beschriebenen Merkmale und Vorteile gelten sinngemäß auch für das weitere Knochenimplantat.

### KURZBESCHREIBUNG DER ZEICHNUNGEN

Weitere Vorteile und Aspekte der Erfindung ergeben sich aus den Ansprüchen und aus der nachfolgenden Beschreibung von bevorzugten Ausführungsbeispielen der Erfindung, die nachfolgend anhand der Figuren erläutert sind. Die Figuren zeigen schematisch Folgendes:
- Fig. 1:: eine Ausführungsform eines beispielhaften Knochenimplantatsystems,
- Fig. 2:: eine Draufsicht auf eine weitere Ausführungsform eines beispielhaften Knochenimplantatsystems,
- Fig. 3:: eine Ausführungsform eines beispielhaften Knochenimplantats,
- Fig. 4a, 4b: eine Ausführungsform eines Grundkörpers eines erfindungsgemäßen Knochenimplantatsystems,
- Fig. 5: eine weitere Ausführungsform eines beispielhaften Knochenimplantatsystems,
- Fig. 6: eine weitere Ausführungsform eines beispielhaften Knochenimplantats,
- Fig. 7: eine weitere Ausführungsform eines beispielhaften Knochenimplantatsystems,
- Fig. 8: eine weitere Ausführungsform eines beispielhaften Knochenimplantatsystems und
- Fig. 9: eine weitere Ausführungsform eines beispielhaften Knochenimplantats.

### DETAILLIERTE FIGURENBESCHREIBUNG

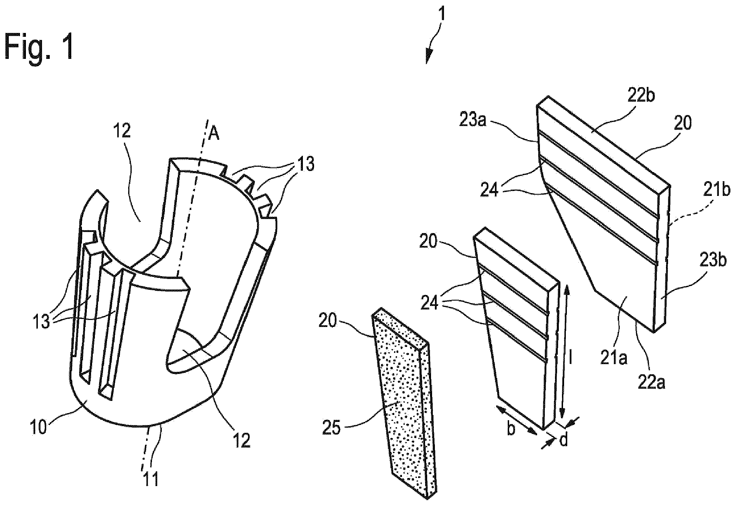

Das in Fig. 1 schematisch dargestellte Knochenimplantatsystem 1 weist einen Grundkörper 10 sowie eine Vielzahl von Formkörpern 20 auf. Dargestellt sind beispielhaft drei Formkörper 20. Es versteht sich, dass das Knochenimplantatsystem 1 aber auch nur zwei oder mehr als drei Formkörper, beispielsweise vier, fünf oder sechs Formkörper, aufweisen kann.

Der Grundkörper 10 ist als ein in axialer Richtung, insbesondere Längsrichtung, A beidseitig offener oder geöffneter konusförmiger Hohlkörper gestaltet.

Der Grundkörper 10 weist ein lasttragendes Material, insbesondere eine Titanlegierung, wie beispielsweise Ti-6Al-4V, oder einen Kunststoff, insbesondere Polyetheretherketon, auf oder besteht aus einem solchen Material. Dadurch ist mit besonderem Vorteil eine ausreichende Sekundärstabilität, d.h. auf knöcherne Anwachsungen beruhende Langzeit-Verankerung, eines durch Zusammenfügen, insbesondere stoffschlüssigem Zusammenfügen, des Grundkörpers 10 sowie der Formkörper 20 erhältlichen Knochenimplantats erzielbar. Bezüglich weiterer geeigneter Materialien für den Grundkörper 10 sei auf die allgemeine Beschreibung verwiesen. Vorzugsweise weist der Grundkörper 10 eine Wandung 11 mit zwei vorzugsweise gegenüberliegend ausgebildeten Durchbrüchen oder Aussparungen 12 auf. Die Durchbrüche bzw. Aussparungen 12 sind vorzugsweise u-förmig gestaltet.

Die Formkörper 20 sind derart mit dem Grundkörper 10 verbindbar, dass sie in radialer Richtung oder außenseitig vom Grundkörper 10 abstehen oder abragen.

Hierzu weist der Grundkörper 10 vorzugsweise Nuten 13 zum formschlüssigen Einsetzen der Formkörper 20 auf. Die Nuten 20 erstrecken sich entlang der Längsachse A des Grundkörpers 10.

Die Formkörper 20 können ein in vivo abbaubares/in vivo resorbierbares Material, wie beispielsweise β-Tricalciumphosphat, aufweisen oder aus einem solchen Material bestehen.

Dadurch lässt sich mit besonderem Vorteil eine biologische Rekonstruktion eines Knochendefekts erzielen. Durch die biologische Rekonstruktion des Knochendefekts kommt es weiter vorteilhafterweise zu einer Minimierung der Knochendefektgröße. Bezüglich weiterer geeigneter Materialien für die Formkörper 20 sei auf die allgemeine Beschreibung verwiesen.

Aufgrund der Vielzahl von Formkörpern 20 besitzt das Knochenimplantatsystem 1 eine modulare Zusammensetzung. Dadurch kann in einer flexibleren Art und Weise patientenspezifischen und mithin unterschiedlichen Knochendefekten entsprochen werden.

Die Formkörper 20 sind vorzugsweise als Flächengebilde, insbesondere in Form von Platten oder Scheiben, gestaltet. Dies bedeutet, dass die Formkörper eine Länge I und eine Breite b aufweisen, die größer sind als eine Dicke d (Höhe) der Formkörper. Bevorzugt besitzen die Formkörper 20 zwei gegenüberliegend angeordnete Hauptflächen 21a und 21b, welche jeweils von zwei gegenüberliegend angeordneten Querseitenflächen 22a und 22b sowie von zwei gegenüberliegend angeordneten Längsseitenflächen 23a und 23b begrenzt sind.

Die Formkörper 20 können wenigstens teilweise Sollbruchlinien 24 aufweisen. Die Sollbruchlinien 24 können sich dabei, wie in Fig. 1 beispielhaft dargestellt, in Querrichtung der Formkörper 20 erstrecken. Alternativ können die Formkörper jedoch auch sich in Längsrichtung der Formkörper erstreckende Sollbruchlinien aufweisen (nicht dargestellt).

Die Sollbruchlinien 24 erlauben eine gezielte Anpassung der Formkörper 20 an einen zu behandelnden oder zu versorgenden Knochendefekt. Dadurch kann die Modularität und mithin die Verwendungsmöglichkeit des erfindungsgemäßen Knochenimplantatsystems 1 zusätzlich erhöht werden.

Grundsätzlich können die Formkörper 20 gleich ausgebildet sein.

Allerdings kann es bevorzugt sein, dass die Formkörper 20 oder ein Teil der Formkörper 20 verschieden, insbesondere in Bezug auf Form und/oder Geometrie, beispielsweise in Bezug auf Länge I und/oder Breite b und/oder Dicke d (Höhe), und/oder Größe, ausgebildet sind. Dies erhöht den modularen Charakter des Knochenimplantatsystems 1 zusätzlich.

Beispielsweise kann es vorgesehen sein, dass die Formkörper 20 oder ein Teil davon eine offenporige und/oder mikrostrukturierte Oberfläche 25 aufweisen. Dies begünstigt die Osteointegration der (betreffenden) Formkörper 20, wodurch die Voraussetzungen für eine verbesserte Sekundärstabilität eines Knochenimplantats geschaffen werden, welches durch Zusammenfügen des Grundkörpers 10 sowie der Formkörper 20 erhältlich ist.

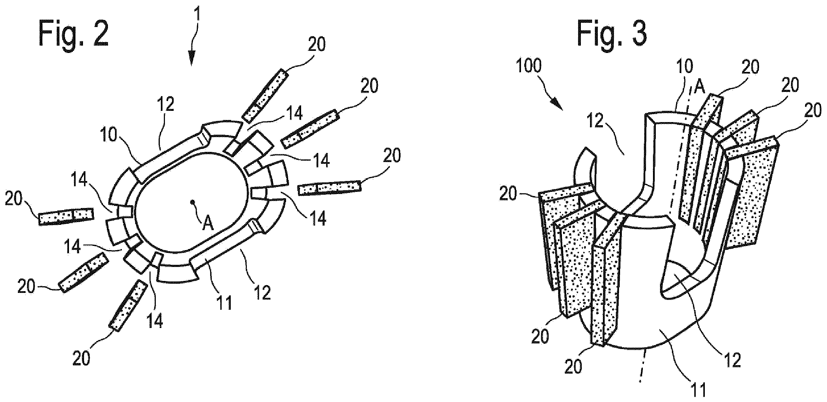

Fig. 2 zeigt schematisch eine Draufsicht auf eine weitere Ausführungsform eines beispielhaften Knochenimplantatsystems 1.

Das Knochenimplantatsystem 1 weist einen Grundkörper 10 sowie eine Vielzahl von Formkörpern 20 auf. Der Grundkörper 10 liegt in Form eines in axialer Richtung, insbesondere Längsrichtung, A beidseitig offenen oder geöffneten, konusförmigen Hohlkörpers vor.

Die Formkörper 20 sind derart mit dem Grundkörper 10 verbindbar, dass die Formkörper 20 in radialer Richtung oder außenseitig von dem Grundkörper abragen. Hierzu weist der Grundkörper Schlitze, d.h. Spalten oder Längsöffnungen, 14 zum formschlüssigen Aufnehmen der Formkörper 20 auf. Die Schlitze 14 erstrecken sich dabei vorzugsweise entlang der Längsachse A des Grundkörpers 10.

Die Formkörper 20 sind jeweils bevorzugt als Flächengebilde, insbesondere plattenförmige Flächengebilde, gestaltet. Die Formkörper 20 sind vorzugsweise offenporös gestaltet. Beispielhaft sind sechs Formkörper 20 dargestellt.

Im Übrigen besitzen die Bezugsziffern der Fig. 2 die gleiche Bedeutung wie die entsprechenden Bezugsziffern der Fig. 1. Bezüglich weiterer Merkmale und Vorteile des in Fig. 2 dargestellten Knochenimplantatsystems 1 wird daher vollständig auf die Beschreibung zur Fig. 1 Bezug genommen. Die dort in Bezug auf den Grundkörper 10 sowie die Formkörper 20 beschriebenen Merkmale und Vorteile gelten sinngemäß auch für das in Fig. 2 dargestellte Knochenimplantatsystem 1.

Fig. 3 zeigt schematisch eine Ausführungsform eines beispielhaften Knochenimplantats 100. Das Knochenimplantat 100 ist durch formschlüssiges Verbinden des in Fig. 2 dargestellten Grundkörpers 10 sowie der in Fig. 2 dargestellten Formkörper 20 hergestellt oder herstellbar. Die Formkörper 20 sind formschlüssig in die Schlitze 14 aufgenommen, so dass die Formkörper 20 in radialer Richtung vom Grundkörper 10 abstehen.

Die in der Fig. 3 im Übrigen dargestellten Bezugsziffern haben die gleiche Bedeutung wie die entsprechenden Bezugsziffern der Fig. 1 und 2. Bezüglich weiterer Merkmale und Vorteile des Knochenimplantats 100 wird daher vollständig auf die Beschreibungen zur Fig. 1 und 2 Bezug genommen. Die dort in Bezug auf den Grundkörper 10 sowie die Formkörper 20 beschriebenen Merkmale und Vorteile gelten sinngemäß auch für das in Fig. 3 dargestellte Knochenimplantat 100.

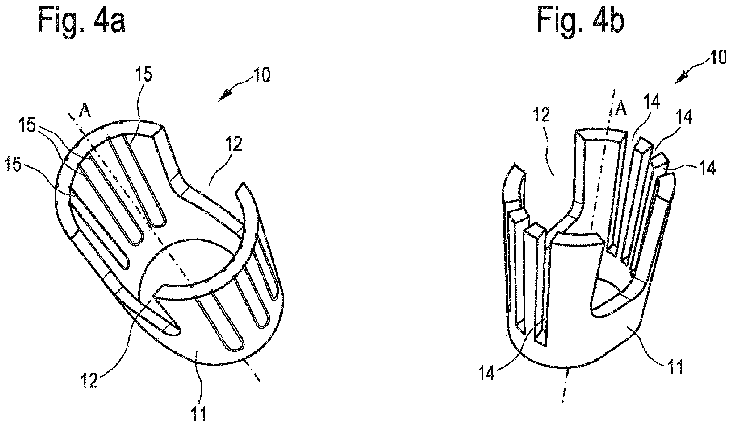

Die Fig. 4a und 4b zeigen schematisch jeweils eine Ausführungsform eines Grundkörpers 10 eines erfindungsgemäßen Knochenimplantatsystems oder Knochenimplantats. Der Grundkörper 10 ist als ein in axialer Richtung, insbesondere Längsrichtung, A beidseitig offener oder geöffneter und vorzugsweise konusförmiger Hohlkörper gestaltet.

Der Grundkörper 10 gemäß Fig. 4a weist Sollbruchlinien 15 auf, welche nach einem Brechen Schlitze, d.h. Spalten oder Längsöffnungen, 14 freigeben, welche sich entlang einer Längsachse A des Grundkörpers 10 erstrecken (siehe Fig. 4b).

Durch die Schlitze 14 können erfindungsgemäße Formkörper formschlüssig von dem Grundkörper 10 aufgenommen werden (siehe auch Fig. 3).

Die übrigen Bezugsziffern der Fig. 4a sowie 4b besitzen dieselben Bedeutungen wie die entsprechenden Bezugsziffern in den vorangegangenen Figuren. Bezüglich weiterer Merkmale und Vorteile des in den Fig. 4a und 4b dargestellten Grundkörpers 10 wird daher auf die entsprechenden Ausführungen zu den Fig. 1 bis 3 Bezug genommen. Die dort in Bezug auf den Grundkörper 10 beschriebenen Merkmale und Vorteile gelten sinngemäß auch für den in den Fig. 4a und 4b dargestellten Grundkörper 10.

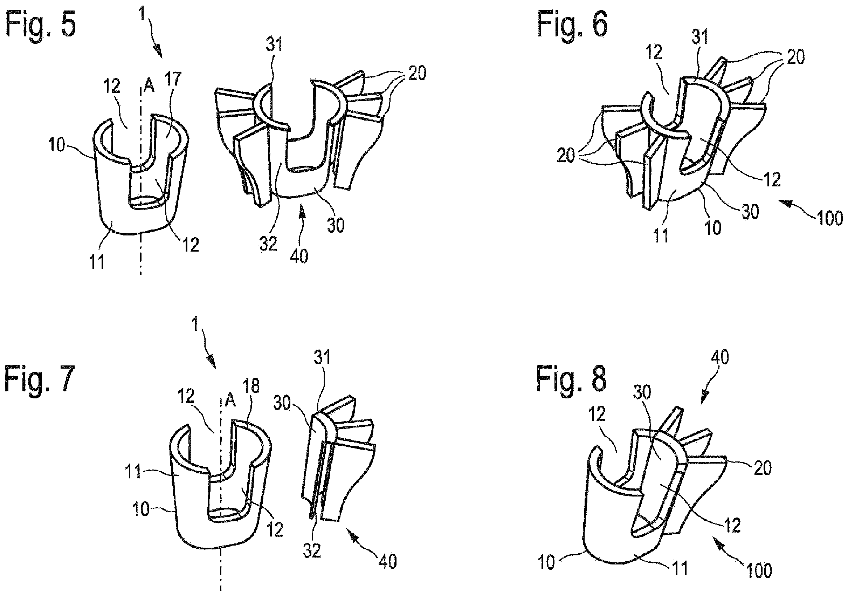

Fig. 5 zeigt eine weitere Ausführungsform eines beispielhaften Knochenimplantatsystems 1.

Das Knochenimplantatsystem 1 weist einen Grundkörper 10, eine Vielzahl von Formkörpern 20 sowie einen Trägerkörper 30 auf.

Der Grundkörper 10 ist als ein in axialer Richtung, vorzugsweise Längsrichtung, A beidseitig geöffneter, konusförmiger Hohlkörper gestaltet.

Der Trägerkörper 30 ist ebenfalls als ein in axialer Richtung, vorzugsweise Längsrichtung, A beidseitig offener oder geöffneter, konusförmiger Hohlkörper gestaltet. Insbesondere ist der Trägerkörper 30 formkomplementär zu dem Grundkörper 10, insbesondere zu einer Innenkontur 17 des Grundkörpers 10, gestaltet.

Die Formkörper 20 sowie der Trägerkörper 30 sind unter Ausbildung eines Klammerkörpers 40 miteinander verbunden, vorzugsweise stoffschlüssig. Bevorzugt ist der Klammerkörper 40 in axialer Richtung A auf eine Wandung 11 des Grundkörpers 10 aufsteckbar.

Der Trägerkörper 30 oder Klammerträger 40 weist ferner einen Kragenabschnitt 31 auf, an welchem die Formkörper 20 stoffschlüssig angefügt sind. Der Kragenabschnitt 31 erstreckt sich dabei in Umfangsrichtung des Trägerkörpers 30 oder Klammerkörpers 40 und kragt radial aus. Dies bewirkt eine Beabstandung der Formkörper 20 von einer Außenfläche 32 des Trägerkörpers 30.

Bevorzugt ist der Klammerkörper 40 im Längsschnitt u-förmig gestaltet.

Die übrigen Bezugsziffern der Fig. 5 besitzen dieselben Bedeutungen wie die entsprechenden Bezugsziffern in den vorangegangenen Figuren. Bezüglich weiterer Merkmale und Vorteile des in Fig. 5 dargestellten Knochenimplantatsystems 1 wird daher auf die entsprechenden Ausführungen zu den vorangegangenen Figuren Bezug genommen. Die dort insbesondere in Bezug auf den Grundkörper 10 sowie die Formkörper 20 beschriebenen Merkmale und Vorteile gelten sinngemäß auch für das in Fig. 5 dargestellte Knochenimplantatsystem 1.

Fig. 6 zeigt schematisch ein Knochenimplantat 100, welches durch formschlüssiges Zusammenfügen des in Fig. 5 dargestellten Grundkörpers 10 sowie des in Fig. 5 dargestellten Klammerkörpers 40, welcher den Trägerkörper 30 sowie die Formkörper 20 aufweist oder aus dem Trägerkörper 30 und den Formkörpern 20 besteht, hergestellt oder herstellbar ist.

Im Übrigen haben die Bezugsziffern der Fig. 6 die gleiche Bedeutung wie die entsprechenden Bezugsziffern der Fig. 5. Bezüglich weiterer Merkmale und Vorteile des Knochenimplantats 100, insbesondere des Grundkörpers 10, der Formkörper 20, des Trägerkörpers 30 sowie des Klammerkörpers 40, wird daher vollständig auf die Beschreibung zur Fig. 5 (sowie zu den vorangegangenen Figuren) Bezug genommen.

Fig. 7 zeigt eine weitere Ausführungsform eines beispielhaften Knochenimplantatsystems 1.

Das Knochenimplantatsystem 1 weist einen Grundkörper 10, einen Trägerkörper 30 sowie eine Vielzahl von Formkörpern 20 auf. Beispielhaft sind drei Formkörper 20 dargestellt. Es versteht sich jedoch, dass das Knochenimplantatsystem 1 auch nur zwei Formkörper oder mehr als drei Formkörper aufweisen kann.

Die Formkörper 20 sowie der Trägerkörper 30 sind unter Ausbildung eines Klammerkörpers 40 miteinander verbunden, vorzugsweise stoffschlüssig. Bevorzugt ist der Klammerkörper 40 in axialer Richtung A auf eine Wandung 11 des Grundkörpers 10 aufsteckbar.

Der Trägerkörper 30 ist formkomplementär zu einem Teil des Grundkörpers 10, insbesondere zu einem Innenkonturabschnitt 18 des Grundkörpers 10, gestaltet. Somit ist der Klammerkörper 40 wenigstens abschnittsweise, insbesondere nur abschnittsweise, formkomplementär zu dem Grundkörper 10, insbesondere einer Innenkontur 18 des Grundkörpers 10, gestaltet.

Der Trägerkörper 30 oder der Klammerkörper 40 weist ferner einen Kragenabschnitt 31 auf, an welchem die Formkörper 20 stoffschlüssig angefügt sind. Der Kragenabschnitt 31 erstreckt sich dabei in Umfangsrichtung des Trägerkörpers 30 bzw. Klammerkörpers 40 und kragt radial aus. Dadurch weisen die Formkörper 20 einen Abstand zu einer Außenfläche 32 des Trägerkörpers 30 auf. Insbesondere ist der Klammerkörper 40 im Längsschnitt u-förmig gestaltet.

Im Übrigen besitzen die Bezugsziffern der Fig. 7 die gleiche Bedeutung wie die entsprechenden Bezugsziffern der Fig. 5 und 6. Bezüglich weiterer Merkmale und Vorteile des in Fig. 7 dargestellten Knochenimplantatsystems 1 wird daher vollständig auf die Beschreibung zu den Fig. 5 und 6 sowie zu den vorangegangenen Figuren Bezug genommen.

Fig. 8 zeigt eine weitere beispielhafte Ausführungsform eines Knochenimplantats 100, welches durch formschlüssiges Zusammenfügen des in Fig. 7 dargestellten Grundkörpers 10 sowie des in Fig. 7 dargestellten Klammerkörpers 40, welcher den Trägerkörper 30 sowie die Formkörper 20 aufweist oder aus dem Trägerkörper 30 und den Formkörpern 20 besteht, erhältlich ist.

Im Übrigen entsprechen die Bezugsziffern der Fig. 8 den entsprechenden Bezugsziffern der Fig. 7. Bezüglich weiterer Merkmale und Vorteile des Knochenimplantats 100, insbesondere des Grundkörpers 10, der Formkörper 20, des Trägerkörpers 30 sowie des Klammerkörpers 40, wird daher vollständig auf die Beschreibung zur Fig. 7 (sowie zu den vorangegangenen Figuren) Bezug genommen.

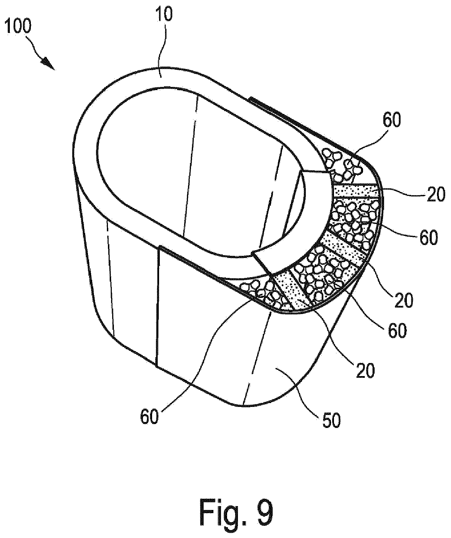

Fig. 9 zeigt eine weitere beispielhafte Ausführungsform eines Knochenimplantats 100. Das Knochenimplantat 100 weist einen Grundkörper 10, eine Vielzahl von Formkörpern 20, eine Abstützstruktur 50 sowie ein Knochenersatzmaterial 60 auf. Das Knochenimplantat 100 ist ausgehend von einem Knochenimplantatsystem, das die vorgenannten Komponenten, insbesondere räumlich oder physikalisch voneinander getrennt, aufweist, erhältlich.

Der Grundkörper 10 ist als ein in axialer Richtung, insbesondere Längsrichtung, beidseitig offener oder geöffneter konusförmiger Hohlkörper gestaltet. Die Formkörper 20 ragen in radialer Richtung oder außenseitig vom Grundkörper 10 ab.

Die Abstützstruktur 50 ist an gegenüberliegenden Außenseiten des Grundkörpers 10, insbesondere stoffschlüssig und/oder formschlüssig und/oder kraftschlüssig, mit dem Grundkörper 10 verbunden, wobei die Abstützstruktur 50 oder eine Innenfläche davon radial von der Grundstruktur 10 oder einer Außenfläche davon beabstandet ist. Die Formkörper 20 sind zwischen der Grundstruktur 10 und der Abstützstruktur 50 angeordnet. Vorzugsweise liegen die Formkörper 20 an der Innenfläche der Abstützstruktur 50 an. Alternativ oder in Kombination können die Formkörper 20 mit der Innenfläche der Abstützstruktur 50, insbesondere stoffschlüssig und/oder formschlüssig und/oder kraftschlüssig, verbunden sein. Durch die Außenfläche des Grundkörpers 10, die Innenfläche der Abstützstruktur 50 sowie die dazwischen angeordneten Formkörper 20 werden eine Vielzahl von Hohlräumen oder Kavitäten definiert, die mit dem Knochenersatzmaterial 60 befüllt sind. Dadurch ist vorteilhafterweise ein hoher Füllungsgrad des zu behandelnden oder versorgenden Knochendefekts, insbesondere unabhängig von der Komplexität der Knochendefektmorphologie, erzielbar.

Das in der Fig. 9 gezeigte Knochenimplantat eignet sich insbesondere zur Behandlung oder Versorgung komplexer Knochendefekte, wie beispielsweise von nicht umschlossenen (uncontained) Randdefekten von Tibia oder Femur. Durch die Verwendung von in vivo abbaubaren/in vivo resorbierbaren Materialien insbesondere für die Formkörper und/oder die Abstützstruktur ist eine biologische Rekonstruktion der Knochendefekte erzielbar. Durch die Verwendung eines Knochenersatzmaterials, das vorzugsweise ebenfalls in vivo abbaubar/in vivo resorbierbar ist, ist zudem vorteilhafterweise eine langfristige Verkleinerung von Knochendefektvolumina durch Umwandlung des Knochenersatzmaterials in Knochen erzielbar.

Bezüglich weiterer Merkmale und Vorteile des Knochenimplantats 100, insbesondere des Grundkörpers 10, der Formkörper 20, der Abstützstruktur 50 sowie des Knochenersatzmaterials 60, wird vollständig auf die Beschreibung zu den vorangegangenen Figuren sowie auf die allgemeine Beschreibung Bezug genommen.

## Patentansprüche

1. Knochenimplantatsystem (1), aufweisend
- einen Grundkörper (10), aufweisend oder bestehend aus einem lasttragenden Material, und
- eine Vielzahl von Formkörpern (20) und/oder eine Abstützstruktur (50), wobei der Grundkörper (10) als ein in axialer Richtung A beidseitig offener Hohlkörper gestaltet ist und die Formkörper (20) derart mit dem Grundkörper (10) verbindbar sind, dass die Formkörper (20) in radialer Richtung von dem Grundkörper (10) abstehen, und/oder die Abstützstruktur (50) derart mit dem Grundkörper (10) verbindbar ist, dass die Abstützstruktur (50) radial von dem Grundkörper (10) beabstandet ist;
**dadurch gekennzeichnet, dass** der
Grundkörper (10) sich entlang der Längsachse des Grundkörpers (10) erstreckende Sollbruchlinien (15) aufweist, welche nach einem Brechen sich entlang der Längsachse des Grundkörpers (10) erstreckende Schlitze (14) freigeben.

2. Knochenimplantatsystem (1) nach Anspruch 1, **dadurch gekennzeichnet, dass** der Grundkörper (10) einen eckenlosen, insbesondere kreisförmigen, ovalen oder elliptischen, Querschnitt aufweist.

3. Knochenimplantatsystem (1) nach Anspruch 1 oder 2, **dadurch gekennzeichnet, dass** der Grundkörper (10) konusförmig gestaltet ist und einen sich wenigstens abschnittsweise verjüngenden Innendurchmesser aufweist.

4. Knochenimplantatsystem (1) nach einem der vorhergehenden Ansprüche, **dadurch gekennzeichnet, dass** der Grundkörper (10) und/oder die Formkörper (20) eine poröse, insbesondere offenporige, und/oder mikrostrukturierte Oberfläche (25) aufweisen/aufweist.

5. Knochenimplantatsystem (1) nach Anspruch 4, **dadurch gekennzeichnet, dass** die Oberfläche (25) von einer Beschichtung des Grundkörpers (10) gebildet ist.

6. Knochenimplantatsystem (1) nach einem der vorhergehenden Ansprüche, **dadurch gekennzeichnet, dass** es sich bei dem lasttragenden Material um ein in vivo nicht abbaubares/in vivo nicht resorbierbares Material, vorzugsweise ausgewählt aus der Gruppe bestehend aus Metalle wie Titan und/oder Tantal, Legierungen wie Titanlegierungen, Keramiken, Kunststoffe, Polyetherketone Polyetheretherketon, Polyetherketonketon, Polyetheretheretherketon, Polyetheretherketonketon, Polyetherketonetherketonketon, Polyolefine und Kombinationen davon, handelt.

7. Knochenimplantatsystem (1) nach einem der vorhergehenden Ansprüche, **dadurch gekennzeichnet, dass** die Formkörper (20) und/oder die Abstützstruktur (50) ein in vivo abbaubares/in vivo resorbierbares Material aufweisen oder aus einem in vivo abbaubaren/in vivo resorbierbaren Material bestehen, wobei das in vivo abbaubare/in vivo resorbierbare Material vorzugsweise ausgewählt ist aus der Gruppe bestehend aus Metalle, Polyhydroxyalkanoate, Polycaprolacton, Poly-p-dioxanon (Poly-1,4-dioxan-2-on), Polymethylenterephthalat, Calciumphosphate, Calciumsulfate, Calciumoxid (CaO), Calciumhydroxid (Ca(OH)₂), Calciumcarbonat (CaCO₃), Calciumcitrat, Calciumlactat, Calciumacetat, Calciumtartrat, Calciumchlorid (CaCl₂), Magnesiumphosphate, Magnesiumsulfate, Magnesiumhydroxid (Mg(OH)₂), Magnesiumhydroxidcarbonat (beispielsweise als 4 MgCO₃ x Mg(OH)₂ x 5 H₂O), Magnesiumoxid (MgO), Magnesiumcitrate (Mg₃(C₆H₅O₇)₂) oder Mg(C₆H₆O₇)), Calcium-Magnesiumcarbonat (CaMg(CO₃)₂) und Kombinationen davon.

8. Knochenimplantatsystem (1) nach einem der vorhergehenden Ansprüche, **dadurch gekennzeichnet, dass** der Grundkörper (10) Nuten (13) und/oder Schlitze (14) zum formschlüssigen Einsetzen der Formkörper (20) aufweist, wobei sich die Nuten (13) und/oder Schlitze (14) entlang der Längsachse des Grundkörpers (10) erstrecken.

9. Knochenimplantatsystem (1) nach einem der vorhergehenden Ansprüche, **dadurch gekennzeichnet, dass** die Formkörper (20) jeweils wenigstens eine Sollbruchlinie (24) aufweisen, wobei sich die Sollbruchlinien (24) vorzugsweise in Längs- oder Querrichtung der Formkörper (20) erstrecken.

10. Knochenimplantatsystem (1) nach einem der vorhergehenden Ansprüche, **dadurch gekennzeichnet, dass** das Knochenimplanatsystem (1) ferner einen Trägerkörper (30) aufweist, wobei die Formkörper (20) und der Trägerkörper (30) unter Ausbildung eines Klammerkörpers (40) stoffschlüssig miteinander verbindbar oder verbunden sind und der Klammerkörper (40) in axialer Richtung auf eine Wandung (11) des Grundkörpers (10) aufsteckbar ist.

11. Knochenimplantatsystem (1) nach Anspruch 10, **dadurch gekennzeichnet, dass** der Trägerkörper (30) formkomplementär zu dem Grundkörper (10) oder einem Teil davon, insbesondere zu einer Innenkontur (17) oder einem Innenkonturabschnitt (18) des Grundkörpers (10), gestaltet ist.

12. Knochenimplantatsystem (1) nach Anspruch 10 oder 11, **dadurch gekennzeichnet, dass** der Trägerkörper (30) einen sich in Umfangsrichtung erstreckenden und radial auskragenden Kragenabschnitt (31) aufweist, an welchem die Formkörper (20) stoffschlüssig angefügt sind.

13. Knochenimplantatsystem (1) nach Anspruch 10, **dadurch gekennzeichnet, dass** der Klammerkörper (40) im Längsschnitt u-förmig gestaltet ist.

14. Knochenimplantat (100), hergestellt oder herstellbar aus einem Knochenimplantatsystem (1) nach einem der vorhergehenden Ansprüche und aufweisend
- einen Grundkörper (10), aufweisend oder bestehend aus einem lasttragenden Material, und
- eine Vielzahl von Formkörpern (20) und/oder eine Abstützstruktur (50),
wobei der Grundkörper (10) als ein in axialer Richtung beidseitig offener Hohlkörper gestaltet ist und die Formkörper (20) derart mit dem Grundkörper (10) verbunden sind, dass die Formkörper (20) in radialer Richtung von dem Grundkörper (10) abstehen, und/oder die Abstützstruktur (50) derart mit dem Grundkörper (10) verbunden ist, dass die Abstützstruktur (50) radial von dem Grundkörper (10) beabstandet ist;
**dadurch gekennzeichnet, dass** der
Grundkörper (10) sich entlang der Längsachse des Grundkörpers (10) erstreckende Sollbruchlinien (15) aufweist, welche nach einem Brechen sich entlang der Längsachse des Grundkörpers (10) erstreckende Schlitze (14) freigeben.

## Claims

1. Bone implant system (1) having
- a base body (10), having or consisting of a load-bearing material, and
- a plurality of shaped bodies (20) and/or a support structure (50),
the base body (10) being designed as a hollow body open on both sides in an axial direction A, and the shaped bodies (20) being connectable to the base body (10) in such a way that the shaped bodies (20) protrude in the radial direction from the base body (10), and/or the support structure (50) being connectable to the base body (10) in such a way that the support structure (50) is radially spaced from the base body (10), **characterized in that** the base body (10) has predetermined breaking lines (15) which extend along the longitudinal axis of the base body (10) and which, after being broken, release slits (14) extending along the longitudinal axis of the base body (10).

2. Bone implant system (1) according to Claim 1, **characterized in that** the base body (10) has a cornerless, in particular circular, oval or elliptical cross section.

3. Bone implant system (1) according to Claim 1 or 2, **characterized in that** the base body (10) is conical and has an internal diameter that tapers at least in sections.

4. Bone implant system (1) according to one of the preceding claims, **characterized in that** the base body (10) and/or the shaped bodies (20) have/has a porous, in particular open-pored, and/or microstructured surface (25).

5. Bone implant system (1) according to Claim 4, **characterized in that** the surface (25) is formed by a coating of the base body (10).

6. Bone implant system (1) according to one of the preceding claims, **characterized in that** the load-bearing material is an in vivo non-degradable/in vivo non-resorbable material, preferably chosen from the group consisting of metals such as titanium and/or tantalum, alloys such as titanium alloys, ceramics, plastics, polyether ketones, polyether ether ketone, polyether ketone ketone, polyether ether ether ketone, polyether ether ketone ketone, polyether ketone ether ketone ketone, polyolefins and combinations thereof.

7. Bone implant system (1) according to one of the preceding claims, **characterized in that** the shaped bodies (20) and/or the support structure (50) have/has an in vivo degradable/in vivo resorbable material or consist/consists of an in vivo degradable/in vivo resorbable material, wherein the in vivo degradable/in vivo resorbable material is preferably chosen from the group consisting of metals, polyhydroxy alkanoates, polycaprolactone, poly-p-dioxanone (poly-1,4-dioxan-2-one), polymethylene terephthalate, calcium phosphates, calcium sulfates, calcium oxide (CaO), calcium hydroxide (Ca(OH)₂), calcium carbonate (CaCO₃), calcium citrate, calcium lactate, calcium acetate, calcium tartrate, calcium chloride (CaCl₂), magnesium phosphates, magnesium sulfates, magnesium hydroxide (Mg(OH)₂), magnesium hydroxide carbonate (for example as 4 MgCO₃ x Mg(OH)₂ x 5 H₂O), magnesium oxide (MgO), magnesium citrates (Mg₃(C₆H₅O₇)₂) or Mg (C₆H₆O₇)), calcium magnesium carbonate (CaMg(CO₃)₂) and combinations thereof.

8. Bone implant system (1) according to one of the preceding claims, **characterized in that** the base body (10) has grooves (13) and/or slits (14) for the form-fitting insertion of the shaped bodies (20), wherein the grooves (13) and/or slits (14) extend along the longitudinal axis of the base body (10).

9. Bone implant system (1) according to one of the preceding claims, **characterized in that** the shaped bodies (20) each have at least one predetermined breaking line (24), wherein the predetermined breaking lines (24) preferably extend in the longitudinal or transverse direction of the shaped bodies (20).

10. Bone implant system (1) according to one of the preceding claims, **characterized in that** the bone implant system (1) moreover has a carrier body (30), wherein the shaped bodies (20) and the carrier body (30) are connectable or connected to one another in a cohesively bonded manner to form a clip body (40), and the clip body (40) is able to be plugged onto a wall (11) of the base body (10) in the axial direction.

11. Bone implant system (1) according to Claim 10, **characterized in that** the carrier body (30) is designed to be complementary in shape to the base body (10) or a part thereof, in particular to an inner contour (17) or an inner contour portion (18) of the base body (10).

12. Bone implant system (1) according to Claim 10 or 11, **characterized in that** the carrier body (30) has a collar portion (31) which extends in the circumferential direction and protrudes radially and to which the shaped bodies (20) are joined in a cohesively bonded manner.

13. Bone implant system (1) according to Claim 10, **characterized in that** the clip body (40) is U-shaped in longitudinal section.

14. Bone implant (100), produced or producible from a bone implant system (1) according to one of the preceding claims and having
- a base body (10), having or consisting of a load-bearing material, and
- a plurality of shaped bodies (20) and/or a support structure (50), the base body (10) being designed as a hollow body open on both sides in an axial direction, and the shaped bodies (20) being connected to the base body (10) in such a way that the shaped bodies (20) protrude from the base body (10) in the radial direction, and/or the support structure (50) being connected to the base body (10) in such a way that the support structure (50) is radially spaced from the base body (10), **characterized in that** the base body (10) has predetermined breaking lines (15) which extend along the longitudinal axis of the base body (10) and which, after being broken, release slits (14) extending along the longitudinal axis of the base body (10).

## Revendications

1. Système d'implant osseux (1), présentant
- un corps de base (10), présentant ou constitué par un matériau porteur de charge, et
- une pluralité de corps moulés (20) et/ou une structure de soutien (50),
le corps de base (10) étant conçu sous la forme d'un corps creux ouvert des deux côtés dans la direction axiale A et les corps moulés (20) pouvant être reliés au corps de base (10) de telle sorte que les corps moulés (20) dépassent du corps de base (10) dans la direction radiale, et/ou la structure de soutien (50) pouvant être reliée au corps de base (10) de telle sorte que la structure de soutien (50) est espacée radialement du corps de base (10) ;
**caractérisée en ce que** le corps de base (10) présente des lignes de rupture (15) s'étendant le long de l'axe longitudinal du corps de base (10), qui, après une rupture, libèrent des fentes (14) s'étendant le long de l'axe longitudinal du corps de base (10).

2. Système d'implant osseux (1) selon la revendication 1, **caractérisé en ce que** le corps de base (10) présente une section transversale sans angle, notamment circulaire, ovale ou elliptique.

3. Système d'implant osseux (1) selon la revendication 1 ou 2, **caractérisé en ce que** le corps de base (10) est conçu sous forme conique et présente un diamètre intérieur qui se rétrécit au moins par sections.

4. Système d'implant osseux (1) selon l'une quelconque des revendications précédentes, **caractérisé en ce que** le corps de base (10) et/ou les corps moulés (20) présentent une surface poreuse, notamment à pores ouverts, et/ou microstructurée (25).

5. Système d'implant osseux (1) selon la revendication 4, **caractérisé en ce que** la surface (25) est formée par un revêtement du corps de base (10).

6. Système d'implant osseux (1) selon l'une quelconque des revendications précédentes, **caractérisé en ce que** le matériau porteur de charge consiste en un matériau non dégradable in vivo/non résorbable in vivo, de préférence choisi dans le groupe constitué par les métaux tels que le titane et/ou le tantale, les alliages tels que les alliages de titane, les céramiques, les matières plastiques, les polyéthercétones polyétheréthercétone, polyéthercétonecétone, polyétherétheréthercétone, polyétheréthercétonecétone, polyéthercétoneéthercétonecétone, les polyoléfines et leurs combinaisons.

7. Système d'implant osseux (1) selon l'une quelconque des revendications précédentes, **caractérisé en ce que** les corps moulés (20) et/ou la structure de soutien (50) présentent un matériau dégradable in vivo/résorbable in vivo ou sont constitués par un matériau dégradable in vivo/résorbable in vivo, le matériau dégradable/résorbable in vivo étant de préférence choisi dans le groupe constitué par les métaux, les polyhydroxyalcanoates, la polycaprolactone, la poly-p-dioxanone (poly-1,4-dioxan-2-one), le polyméthylène téréphtalate, les phosphates de calcium, les sulfates de calcium, l'oxyde de calcium (CaO), l'hydroxyde de calcium (Ca(OH)₂), le carbonate de calcium (CaCO₃), le citrate de calcium, le lactate de calcium, l'acétate de calcium, le tartrate de calcium, le chlorure de calcium (CaCl₂), les phosphates de magnésium, les sulfates de magnésium, l'hydroxyde de magnésium (Mg(OH)₂), le carbonate d'hydroxyde de magnésium (par exemple sous la forme 4 MgCO₃ x Mg(OH)₂ x 5 H₂O), l'oxyde de magnésium (MgO), les citrates de magnésium (Mg₃(C₆H₅O₇)₂) ou Mg(C₆H₆O₇)), le carbonate de calcium et de magnésium (CaMg(CO₃)₂) et leurs combinaisons.

8. Système d'implant osseux (1) selon l'une quelconque des revendications précédentes, **caractérisé en ce que** le corps de base (10) présente des rainures (13) et/ou des fentes (14) pour l'insertion par complémentarité de forme des corps moulés (20), les rainures (13) et/ou les fentes (14) s'étendant le long de l'axe longitudinal du corps de base (10).

9. Système d'implant osseux (1) selon l'une quelconque des revendications précédentes, **caractérisé en ce que** les corps moulés (20) présentent chacun au moins une ligne de rupture (24), les lignes de rupture (24) s'étendant de préférence dans la direction longitudinale ou transversale des corps moulés (20).

10. Système d'implant osseux (1) selon l'une quelconque des revendications précédentes, **caractérisé en ce que** le système d'implant osseux (1) présente en outre un corps de support (30), les corps moulés (20) et le corps de support (30) pouvant être reliés ou étant reliés entre eux par liaison de matière en formant un corps d'agrafe (40) et le corps d'agrafe (40) pouvant être enfiché dans la direction axiale sur une paroi (11) du corps de base (10) .

11. Système d'implant osseux (1) selon la revendication 10, **caractérisé en ce que** le corps de support (30) est conçu sous forme complémentaire à celle du corps de base (10) ou d'une partie de celui-ci, notamment à un contour intérieur (17) ou à une section de contour intérieur (18) du corps de base (10).

12. Système d'implant osseux (1) selon la revendication 10 ou 11, **caractérisé en ce que** le corps de support (30) présente une section de collerette (31) s'étendant dans la direction périphérique et faisant saillie radialement, sur laquelle les corps moulés (20) sont assemblés par liaison de matière.

13. Système d'implant osseux (1) selon la revendication 10, **caractérisé en ce que** le corps d'agrafe (40) est conçu sous forme de U en coupe longitudinale.

14. Implant osseux (100), fabriqué ou pouvant être fabriqué à partir d'un système d'implant osseux (1) selon l'une quelconque des revendications précédentes et présentant
- un corps de base (10), présentant ou constitué par un matériau porteur de charge, et
- une pluralité de corps moulés (20) et/ou une structure de soutien (50),
le corps de base (10) étant conçu sous forme de corps creux ouvert des deux côtés dans la direction axiale et les corps moulés (20) étant reliés au corps de base (10) de telle sorte que les corps moulés (20) dépassent du corps de base (10) dans la direction radiale, et/ou la structure de soutien (50) étant reliée au corps de base (10) de telle sorte que la structure de soutien (50) est espacée radialement du corps de base (10) ;
**caractérisé en ce que** le corps de base (10) présente des lignes de rupture (15) s'étendant le long de l'axe longitudinal du corps de base (10), qui, après une rupture, libèrent des fentes (14) s'étendant le long de l'axe longitudinal du corps de base (10).
